(19) Europäisches Patentamt
European Patent Office
Office européen des brevets

(11) **EP 4 393 414 B1**

(12) **EUROPEAN PATENT SPECIFICATION**

(45) Date of publication and mention of the grant of the patent:
**17.12.2025 Bulletin 2025/51**

(21) Application number: **22872721.0**

(22) Date of filing: **08.09.2022**

(51) International Patent Classification (IPC):
*A61B 17/115* (2006.01)  *A61L 31/04* (2006.01)
*A61L 31/14* (2006.01)  *A61B 17/072* (2006.01)
*A61B 17/00* (2006.01)  *A61B 17/11* (2006.01)

(52) Cooperative Patent Classification (CPC):
**A61B 17/07292;** A61B 2017/00004;
A61B 2017/1132

(86) International application number:
**PCT/JP2022/033669**

(87) International publication number:
**WO 2023/047966 (30.03.2023 Gazette 2023/13)**

(54) **MEDICAL DEVICE**

MEDIZINISCHE VORRICHTUNG

DISPOSITIF MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2021 JP 2021156611**

(43) Date of publication of application:
**03.07.2024 Bulletin 2024/27**

(73) Proprietor: **TERUMO Kabushiki Kaisha**
**Tokyo 151-0072 (JP)**

(72) Inventors:
• **SUZUKI, Miria**
**Ashigarakami-gun, Kanagawa 259-0151 (JP)**
• **ARAMAKI, Naoki**
**Ashigarakami-gun, Kanagawa 259-0151 (JP)**
• **KAI, Miho**
**Ashigarakami-gun, Kanagawa 259-0151 (JP)**
• **SHIRAISHI, Mizuho**
**Ashigarakami-gun, Kanagawa 259-0151 (JP)**

(74) Representative: **Casalonga**
**Casalonga & Partners**
**Bayerstraße 71/73**
**80335 München (DE)**

(56) References cited:
WO-A1-2019/156230  WO-A1-2020/196857
JP-A- 2013 010 004  JP-A- 2021 049 168
JP-A- 2021 049 168  JP-A- 2021 049 191
JP-A- 2021 049 191  US-A1- 2003 183 671
US-A1- 2005 059 996

**Description**

Technical Field

The present invention relates to a medical device to be used for anastomosis of living body organs. In particular, the present invention relates to medical device comprising:

a sheet-like main body portion in which a plurality of through holes are formed, and which induces expression of biological components by being applied to an anastomosis portion of living body organs and promotes fusion of the anastomosis portion by allowing the induced biological components to penetrate through the through holes and accumulating the induced biological components;
a cylindrical portion that is disposed closer to a center portion side in a plane direction of the main body portion than an outer peripheral portion in the plane direction of the main body portion, protrudes in a first direction intersecting the plane direction of the main body portion, and has a lumen formed therein; and
a connection portion that connects the main body portion and the cylindrical portion,
wherein a relationship among hardness of the cylindrical portion, hardness of the connection portion, and hardness of the main body portion satisfies the following expression (1):

Hardness of cylindrical portion > hardness of connection portion such as it is e.g. known from JP 20121 049191 A1.

Background Art

In the medical field, a procedure of joining living body organs by surgery (for example, anastomosis joining the alimentary canal) is known. It is also known that, in a case where the procedure as described above is performed, it is important as a postoperative prognostic determinant that delay in fusion at a joined portion (hereinafter, also referred to as an "anastomosis portion") where living body organs are joined to each other does not occur.
Various methods and medical tools are used in the procedure of joining living body organs, and for example, a method of suturing living body organs with a biodegradable suture thread and a method of using a mechanical anastomosis device (see Patent Literature 1) for performing anastomosis by a stapler have been proposed. In particular, in a case where anastomosis is performed using a mechanical anastomosis device, it is possible to increase joining force between living body organs at an anastomosis portion as compared with the method using a suture thread, so that it is possible to reduce a risk of suture failure.
However, a degree of progress of fusion at the anastomosis portion also depends on a state of living tissues at a site to be anastomosed of a patient, and the like. Thus, for example, even in a case where an anastomosis device as described in Patent Literature 1 is used, there is a possibility that a risk of suture failure cannot be sufficiently reduced depending on a state of the living tissues of the patient.
In order to cope with the problem as described above, use of a medical device described in Patent Literature 2 below has been proposed in anastomosis in which living body organs are joined.
The medical device described in Patent Literature 2 includes a sheet-like main body portion in which through holes are formed. When a procedure is performed using the medical device, a surgeon sets the sheet-like main body portion in an anastomosis device. Using the anastomosis device, the surgeon indwells the sheet-like main body portion in a state of being put between living body organs to be anastomosed. The sheet-like main body portion promotes fusion of the anastomosis portion by accumulating biological components in the through holes. It is therefore possible to effectively increase joining force at the anastomosis portion by performing anastomosis using the above-described medical device.

Citation List

Patent Literature

Patent Literature 1: JP 2007-505708 A
Patent Literature 2: WO 2019/156230 A

Summary of Invention

Technical Problem

In a case where anastomosis is performed using the above-described medical device, the following problems are assumed.

In some cases, the sheet-like main body portion included in the medical device is preferably configured to have desired flexibility and thinness in order to be able to follow movement of the living body organs in a state of being indwelled in the living body. However, in a case where the sheet-like main body portion is configured as described above, the main body portion is more likely to be easily deformed when the surgeon sets the medical device in the anastomosis device. If the main body portion is deformed, it takes time and effort to set the main body portion in the anastomosis device. Furthermore, it is also conceivable that the main body portion is displaced from the set position of the anastomosis device or falls off from the anastomosis device while the procedure is performed. Furthermore, if excessive force is applied to the main body portion when the surgeon handles the medical device, the main body portion may be damaged.

An object of the present invention is to provide a medical device including a main body portion capable of promoting fusion between living body organs and having improved handleability at the time of use.

Solution to Problem

In order to solve the above problems, the present invention provides a medical device according to independent claim 1. The dependent claims relate to advantageous embodiments.

Advantageous Effects of Invention

According to an embodiment of the present invention, it is possible to provide a medical device including a main body portion capable of promoting fusion between living body organs and having improved handleability at the time of use.

Brief Description of Drawings

Fig. 1 is a perspective view of a medical device according to an embodiment of the present invention.
Fig. 2 is a partial cross-sectional view of a main body portion of the medical device taken along a line 2-2 illustrated in Fig. 1.
Fig. 3 is a plan view of the medical device.
Fig. 4 is a cross-sectional view of the medical device taken along a line 4-4 illustrated in Fig. 3.
Fig. 5 is a partial cross-sectional view for explaining operational effects of the medical device.
Fig. 6 is a partial cross-sectional view for explaining a dimensional example of the medical device.
Fig. 7 is a flowchart indicating procedure of a treatment method using the medical device.
Fig. 8 is a flowchart indicating procedure of an embodiment of a treatment method (large intestine anastomosis).
Fig. 9 is a schematic cross-sectional view for explaining colorectal anastomosis.
Fig. 10 is a schematic cross-sectional view for explaining the colorectal anastomosis.
Fig. 11 is a schematic cross-sectional view for explaining the colorectal anastomosis.
Fig. 12 is a cross-sectional view of a medical device according to a first modification.
Fig. 13 is a cross-sectional view of a medical device according to a second modification.
Fig. 14 is a cross-sectional view of a medical device according to a third modification.
Fig. 15 is a cross-sectional view of a medical device according to a fourth modification.
Fig. 16 is a cross-sectional view of a medical device according to a fifth modification.
Fig. 17 is a cross-sectional view of a medical device according to a sixth modification.
Fig. 18 is a cross-sectional view of a medical device according to a seventh modification.
Fig. 19 is a cross-sectional view of a medical device according to an eighth modification.
Fig. 20 is a cross-sectional view of a medical device according to a ninth modification.
Fig. 21 is a cross-sectional view of a medical device according to a tenth modification.
Fig. 22 is a cross-sectional view of a medical device according to an eleventh modification.

Description of Embodiments

(Embodiment)

Hereinafter, an embodiment of the present invention will be described with reference to the accompanying drawings. In the description of the drawings, the same elements are denoted by the same reference numerals, and redundant description will be omitted. Furthermore, dimensional ratios of the drawings are exaggerated for illustration purpose and may differ from actual ratios.

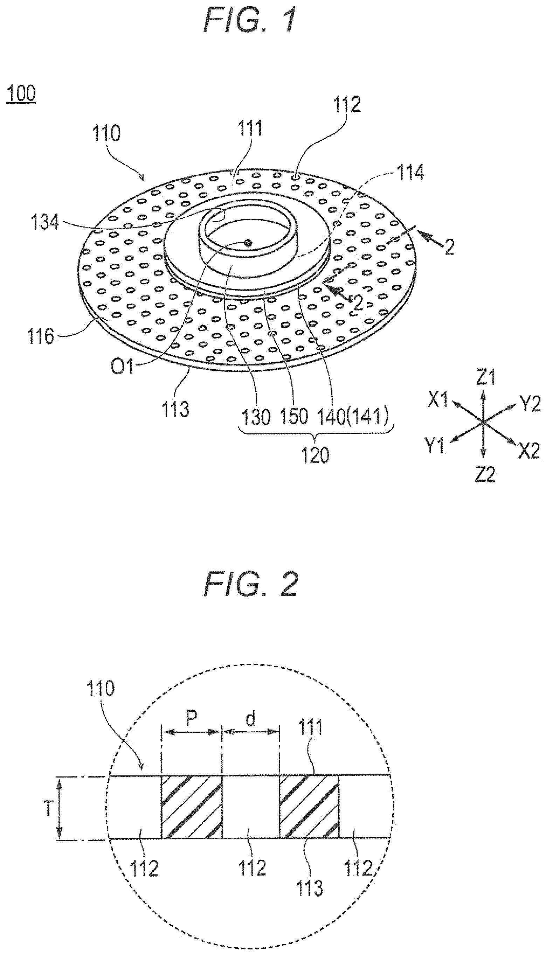

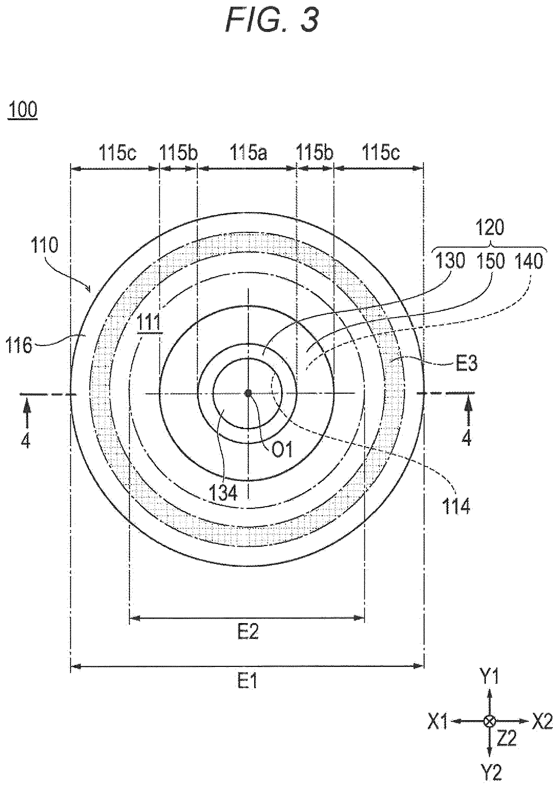

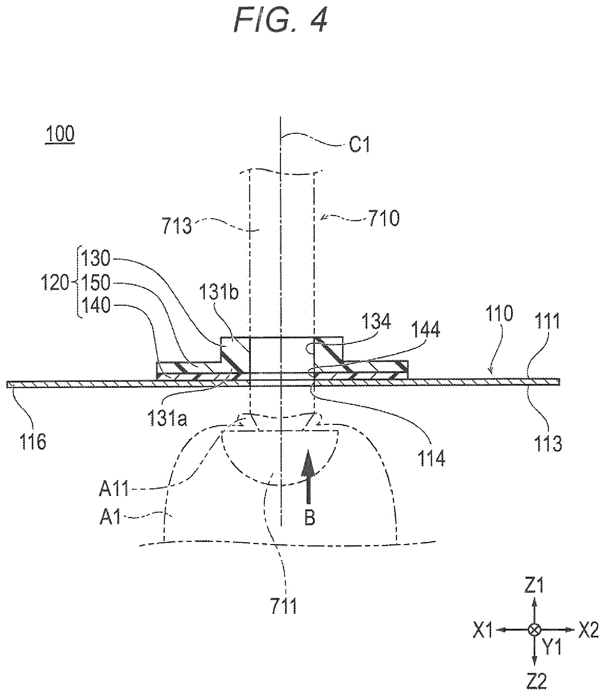

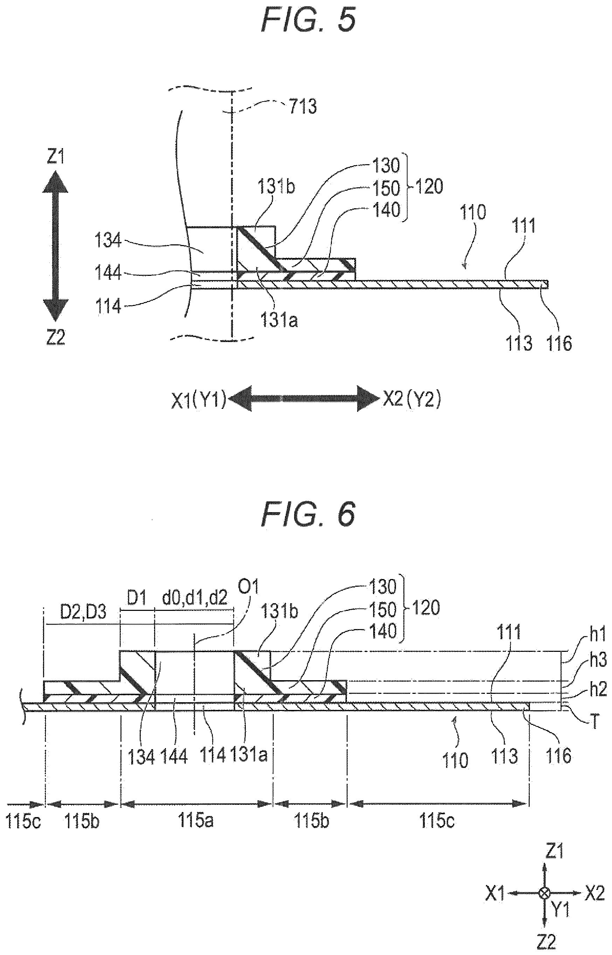

Fig. 1 is a perspective view illustrating a medical device 100 according to the present embodiment. Fig. 2 is an enlarged cross-sectional view illustrating part of a main body portion 110 of the medical device 100 taken along a line 2-2 illustrated in Fig. 1. Fig. 3 is a plan view of the medical device 100 as viewed from a front surface 111 side of the main body portion 110. Fig. 4 is a cross-sectional view of the medical device 100 taken along a line 4-4 illustrated in Fig. 3. Figs. 5 and 6 are enlarged cross-sectional views illustrating part of the medical device 100. Note that in Figs. 3 to 6, through holes 112 of the main body portion 110 are not illustrated.

<Medical device>

As outlined with reference to Figs. 1 and 4, the medical device 100 has a sheet-like main body portion 110 and a fixing portion 120 disposed on the main body portion 110.

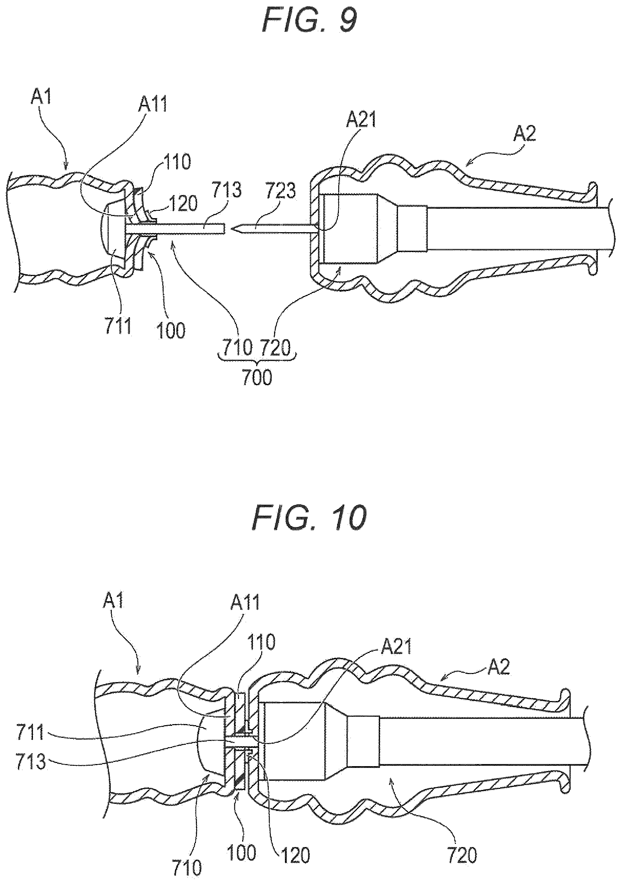

As illustrated in Figs. 9 to 11, the medical device 100 can be applied to a procedure for joining predetermined living body organs (for example, anastomosis of the gastrointestinal tract). As will be described later, in the description of the present specification, large intestine anastomosis will be described as a procedure example using the medical device 100.

In a procedure using the medical device 100, the surgeon indwells at least part of the main body portion 110 of the medical device 100 between two or more living body organs to be joined. The main body portion 110 of the medical device 100 functions as a fusion promoting device that promotes fusion of living tissues of two living body organs.

Specifically, the main body portion 110 is applied to an anastomosis portion of living body organs to induce expression of biological components of the living body organs. The main body portion 110 can promote fusion by the induced biological components penetrating through the through holes 112 and being accumulated.

<Main body portion>

As illustrated in Figs. 1 and 3, the main body portion 110 is formed with a sheet-like member in which a plurality of through holes 112 are formed.

The plurality of through holes 112 penetrate between the front surface 111 of the main body portion 110 and a back surface 113 of the main body portion 110 along a thickness direction (vertical direction in Fig. 2) of the main body portion 110. In the present specification, the same reference numeral 112 is used for "a plurality of through holes" and a "through hole" for convenience of description.

The main body portion 110 can be configured to have a circular shape in plan view illustrated in Fig. 3. However, a planar shape of the main body portion 110 is not particularly limited and may be, for example, an ellipse or a polygon (such as a rectangle and a triangle).

The thickness direction of the main body portion 110 is indicated by an arrow Z1-Z2 in the respective drawings. A plane direction of the main body portion 110 is indicated by an arrow X1-X2 or an arrow Y1-Y2 in the respective drawings.

As illustrated in Fig. 4, the main body portion 110 has a hole portion 114 formed in a predetermined range including a center portion O1 (see Figs. 1 and 3) in the plane direction of the main body portion 110.

The center portion O1 of the main body portion 110 is a rotation center of the main body portion 110 in a case where the main body portion 110 has a rotationally symmetric shape.

A first engagement portion 713 provided in a first engagement tool 710 of a joining device 700 can be inserted into the hole portion 114.

In a procedure using the medical device 100 and the joining device 700, the surgeon can fix (hold) the medical device 100 to the first engagement portion 713 by inserting the first engagement portion 713 of the first engagement tool 710 into the hole portion 114 of the main body portion 110, the lumen 134 of the cylindrical portion 130 which will be described later, and a hole portion 144 of a connection portion 140 (see Figs. 4 and 9).

As illustrated in Fig. 1, the through holes 112 formed in the main body portion 110 are regularly and periodically provided in the plane direction of the main body portion 110. However, the respective through holes 112 may be randomly provided in each portion in the plane direction of the main body portion 110.

As illustrated in Fig. 2, each through hole 112 extends substantially vertically between the front surface 111 and the back surface 113 along the thickness direction (vertical direction in Fig. 2) of the main body portion 110. Each through hole 112 may be bent in a zigzag shape or curved between the front surface 111 and the back surface 113 in a cross section along the thickness direction of the main body portion 110.

Each through hole 112 has a substantially circular planar shape. However, the planar shape of each through hole 112 is not particularly limited and may be, for example, an elliptical shape, a polygonal shape (such as rectangular shape and a triangular shape), an irregular planar shape, or the like. A planar shape or a cross-sectional shape may be different for each through hole 112.

A thickness T (dimension T illustrated in Fig. 2) of the main body portion 110 is not particularly limited, but is preferably 0.05 to 0.3 mm and more preferably 0.1 to 0.2 mm from the viewpoint of preventing damage of the main body portion 110 at the time of handling the medical device 100. In a case where the thickness T of the main body portion 110 is equal to or less than 0.3 mm (particularly equal to or less than 0.2 mm), flexibility of the main body portion 110 can be enhanced. As a result, the main body portion 110 is in close contact with living tissues, and followability with respect to movement of the living tissue is enhanced.

In the main body portion 110, for example, a value of a ratio of a hole diameter d (which is a distance illustrated in Fig.2) of the through hole 112 to a pitch P (which is a distance P illustrated in Fig. 2 and a distance between adjacent through holes 112) of the through holes 112 is preferably equal to or greater than 0.25 and less than 40. Note that in a case where the planar shape of the through hole 112 is a perfect circle, the hole diameter d of the through hole 112 is equal to a diameter of the perfect circle. On the other hand, in a case where the planar shape of the through hole 112 is not a perfect circle, a diameter (equivalent circle diameter) of the perfect circle having the same area as the area of an opening portion of the through hole 112 (a portion facing the front surface 111 or the back surface 113 in the through hole 112) can be set as the hole diameter d of the through hole 112.

The main body portion 110 has the plurality of through holes 112, and thus, there are a plurality of values of the hole diameter d corresponding to the respective through holes 112. Thus, in the present embodiment, in calculating the value of the ratio described above, an arithmetic average value of two or more points of the values of the hole diameter d, respectively corresponding to the plurality of through holes 112 is used as a representative value of the hole diameter **d.** On the other hand, the pitch P of the plurality of through holes 112 is defined by a shortest distance between the opening portions of the two through holes 112. However, as for the value of the pitch P, there are a plurality of values of the pitch P corresponding to combinations of the adjacent through holes 112. Thus, in the present embodiment, in calculating the value of the ratio described above, an arithmetic average value of two or more points of the values of the pitch P, respectively corresponding to the combinations of the adjacent through holes 112, is used as a representative value of the pitch P.

The pitch P, the hole diameter d, the ratio of the hole diameter d to the pitch P, and the like, of the through holes 112 described above are merely examples, and the present invention is not limited thereto.

The main body portion 110 can be formed with, for example, a biodegradable material. The constituent material of the main body portion 110 is not particularly limited, and examples of the material of the main body portion 110 include a biodegradable polymer. As the biodegradable polymer, for example, known biodegradable (co) polymers such as those described in JP 2011-528275 W, JP 2008-514719 W, WO 2008-1952 W, JP 2004-509205 W, and the like, can be used. Specifically, (1) a polymer selected from the group consisting of aliphatic polyester, polyester, polyacid anhydride, polyorthoester, polycarbonate, polyphosphazene, polyphosphoric acid ester, polyvinyl alcohol, polypeptide, polysaccharide, protein, and cellulose; (2) a copolymer composed of one or more monomers constituting the (1) above can be used. In other words, the main body portion 110 preferably contains a biodegradable resin of at least one type of a polymer selected from the group consisting of aliphatic polyester, polyester, polyacid anhydride, polyorthoester, polycarbonate, polyphosphazene, polyphosphoric acid ester, polyvinyl alcohol, polypeptide, polysaccharide, protein, and cellulose, or a copolymer composed of one or more monomers constituting the polymer.

A method for manufacturing the main body portion 110 is not particularly limited, and examples of the method for manufacturing the main body portion 110 can include a method in which fibers formed with the above-described biodegradable polymer are manufactured, and a mesh-shaped sheet is manufactured using the fibers. A method for manufacturing fibers formed with the biodegradable resin is not particularly limited, and examples of the method for manufacturing fibers can include an electrospinning method (electrospinning method/electrostatic spinning method), a melt blowing method, and the like. The main body portion 110 may be manufactured by selecting and using only one of the above methods or by appropriately combining and using two or more of the above methods. As still another example of the method for manufacturing the main body portion 110, a method in which the biodegradable sheet according to the present invention is manufactured by spinning fibers formed with the biodegradable resin described above according to a conventional method and knitting the obtained fibers into a mesh shape, a method in which the biodegradable sheet is manufactured by compressing the fibers, and a method in which the biodegradable sheet is manufactured by entangling the fibers without weaving the fibers, can be used.

The main body portion 110 causes biological reaction with a constituent material such as a biodegradable polymer. The main body portion 110 induces expression of biological components such as fibrin by this action. The biological components induced in this way are allowed to penetrate through the through holes 112 of the main body portion 110 and are accumulated, thereby promoting fusion. Thus, by disposing the main body portion 110 between the living body organs to be joined, fusion is promoted by the above mechanism.

Note that a material of the main body portion 110 may not be biodegradable as long as fusion can be promoted.

The hole portion 114 of the main body portion 110 has a hole diameter d0 (see Fig. 6) larger than each through hole 112.

The hole portion 114 can be configured to have a circular planar shape. The hole portion 114 can be formed to have a hole diameter d0 of, for example, 5 mm to 25 mm. The planar shape of the hole portion 114 is not particularly limited and may be, for example, an ellipse or a polygon (such as a rectangle and a triangle). A size of the hole portion 114 is also not particularly limited.

As illustrated in Fig. 6, the medical device 100 is disposed so that the hole portion 114 of the main body portion 110, the lumen 134 of the cylindrical portion 130, and the hole portion 144 of the connection portion 140 overlap each other in the plane direction of the main body portion 110. The hole diameter d0 of the hole portion 114 of the main body portion 110 is substantially equal to an inner diameter d1 of the cylindrical portion 130 and a hole diameter d2 of the hole portion 144 of the connection portion 140.

Note that, in the medical device 100, the hole diameter d0 of the hole portion 114 of the main body portion 110, the inner diameter d1 of the lumen 134 of the cylindrical portion 130, and the hole diameter d2 of the hole portion 144 of the connection portion 140 do not have to be equal as long as the first engagement portion 713 of the first engagement tool 710 can be inserted. In addition, the hole portion 114 of the main body portion 110, the lumen 134 of the cylindrical portion 130, and the hole portion 144 of the connection portion 140 do not have to be arranged so as to overlap over an entire range at the same position in the plane direction of the main body portion 110.

The hole portion 114 may be prepared in advance in the main body portion 110 or may be prepared by the surgeon while performing anastomosis. Furthermore, the surgeon can select various modifications regarding a shape, a structure, and the like, of the main body portion 110 according to progress of the procedure, and the like.

With reference to a plan view of Fig. 3, a positional relationship between each part of the medical device 100 and each part of the joining device 700 to be used for anastomosis will be described.

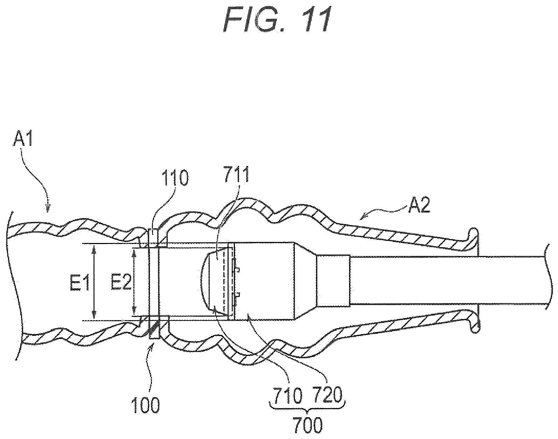

A first region E1 has, in at least part of the first region E1, a region where the first engagement tool 710 and the second engagement tool 720 of the joining device 700 overlap each other (see Fig. 11). Note that the first region E1 may be larger than an outer diameter of the joining device 700 (maximum outer diameter of the first engagement tool 710 and the second engagement tool 720).

The second region E2 is a portion punched out by the joining device 700 in order to form an opening portion communicating with the lumen of the living body organs (for example, large intestine) to be joined in the main body portion 110.

Each of the engagement tools 710 and 720 included in the joining device 700 includes a cutter (blade). When joining the living body organs, the joining device 700 engages the engagement tools 710 and 720 with each other and punches out part of the main body portion 110 at the same time as suturing (see Figs. 10 and 11).

In the medical device 100, a fixing portion 120 is disposed in a range including the center portion O1 of the main body portion 110. Further, the fixing portion 120 is disposed in a range that falls within the center portion O1 side of the second region E2 that is a punching target range. Thus, when the anastomosis portion is formed using the joining device 700, the fixing portion 120 is punched out and cut out together with part of the main body portion 110 (see Fig. 11).

A third region E3 (shaded portion in Fig. 3) corresponds to a joined portion that is indwelled in a state of being put between the living body organs when the living body organs are joined to each other by the engagement tools 710 and 720. The third region E3 is indwelled between the living body organs to promote fusion of the anastomosis portion. In the procedure using the joining device 700, a joining member (for example, staple) is supplied to the third region E3, and the third region E3 is joined to the living body organs.

In a case where the joining device 700 is constituted with a known automatic anastomosis device, the first engagement tool 710 may be constituted with, for example, an anvil including a head portion 711 and a first engagement portion 713 (shaft) connected to the head portion 711 (see Fig. 4).

The second engagement tool 720 can be constituted with, for example, a trocar including a second engagement portion 723 (engagement pin) engageable with the first engagement portion 713 (see Figs. 10 and 11).

As the joining device 700, for example, a known circular stapler can be used. However, a specific structure, type, and the like, of the joining device 700 are not particularly limited.

<Fixing portion>

As illustrated in Figs. 1, 4, and 5, the fixing portion 120 includes the cylindrical portion 130, the connection portion 140, and the edge portion 150.

As illustrated in Figs. 4 and 9, the fixing portion 120 has a function of increasing fixing force (holding force) of the medical device 100 with respect to the first engagement portion 713 in a state where the medical device 100 is set in the first engagement tool 710.

The cylindrical portion 130 is disposed closer to the center portion O1 side in the plane direction of the main body portion 110 than an outer peripheral portion 116 in the plane direction of the main body portion 110.

The above-described "outer peripheral portion 116 of the main body portion 110" can be defined in an arbitrary range on an outer peripheral side of the third region E3 from an outer peripheral edge of the main body portion 110 toward the center portion O1 side.

The cylindrical portion 130 protrudes in a first direction intersecting the plane direction of the main body portion 110. The lumen 134 through which the first engagement portion 713 of the first engagement tool 710 can be inserted is formed inside the cylindrical portion 130.

The above-described "first direction" is the same direction as the thickness direction (vertical direction in Figs. 4, 5, and 6) of the main body portion 110 in a no-load state where no external force is applied. The above "first direction" is synonymous with "axial direction" (direction along an axis C1 indicated in Fig. 4) in which the cylindrical portion 130 extends. Hereinafter, the "first direction" is also referred to as an "axial direction".

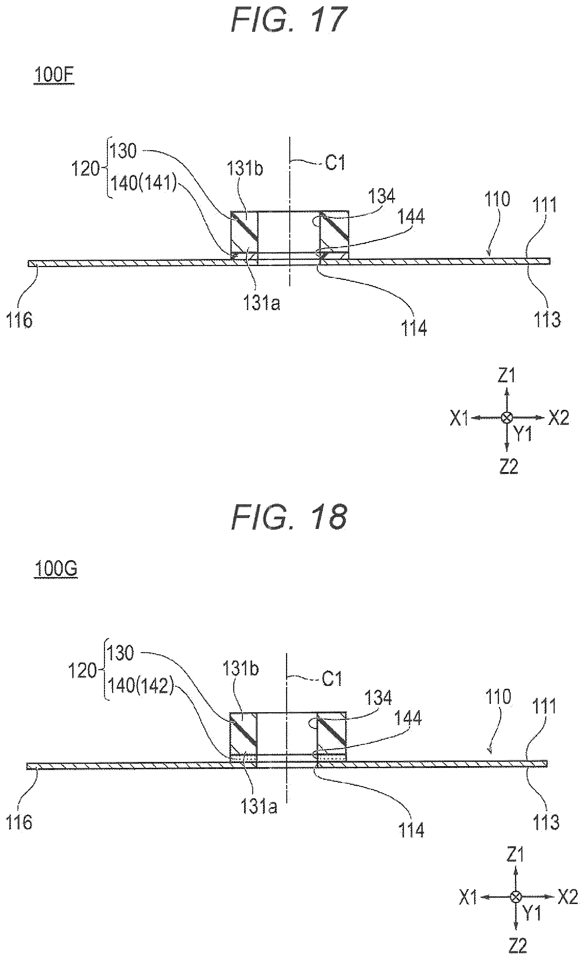

As illustrated in Fig. 4, the lumen 134 of the cylindrical portion 130 extends in substantially the same cross-sectional shape along the axial direction of the cylindrical portion 130. However, as described in each modification which will be described later, the cross-sectional shape of the lumen 134 of the cylindrical portion 130 does not have to be a constant shape along the axial direction of the cylindrical portion 130 (see Figs. 18 to 21).

As illustrated in Fig. 3, the planar shape of the outer peripheral portion of the cylindrical portion 130 and the lumen 134 can be formed in, for example, a circular shape. However, the planar shape of each portion is not particularly limited as long as the first engagement portion 713 can be inserted through the cylindrical portion 130 and may be, for example, an elliptical shape, a polygonal shape, or the like. Furthermore, the outer peripheral portion of the cylindrical portion 130 and the lumen 134 do not have to have similar shapes and may have different planar shapes, for example.

The cylindrical portion 130 has one end portion 131a located on the connection portion 140 side (main body portion 110 side) and the other end portion 131b located on the opposite side of the one end portion 131a.

The one end portion 131a of the cylindrical portion 130 is opened facing the hole portion 144 of the connection portion 140. The other end portion 131b of the cylindrical portion 130 is opened facing the outside on the opposite side to the portion where the connection portion 140 is located.

In the procedure using the medical device 100, the first engagement portion 713 of the first engagement tool 710 can be inserted into the lumen 134 of the cylindrical portion 130 from the one end portion 131a side toward the other end portion 131b side. An insertion direction of the first engagement portion 713 is indicated by an arrow B in Fig. 4.

As illustrated in Figs. 1, 4, and 5, the connection portion 140 connects the main body portion 110 and the cylindrical portion 130.

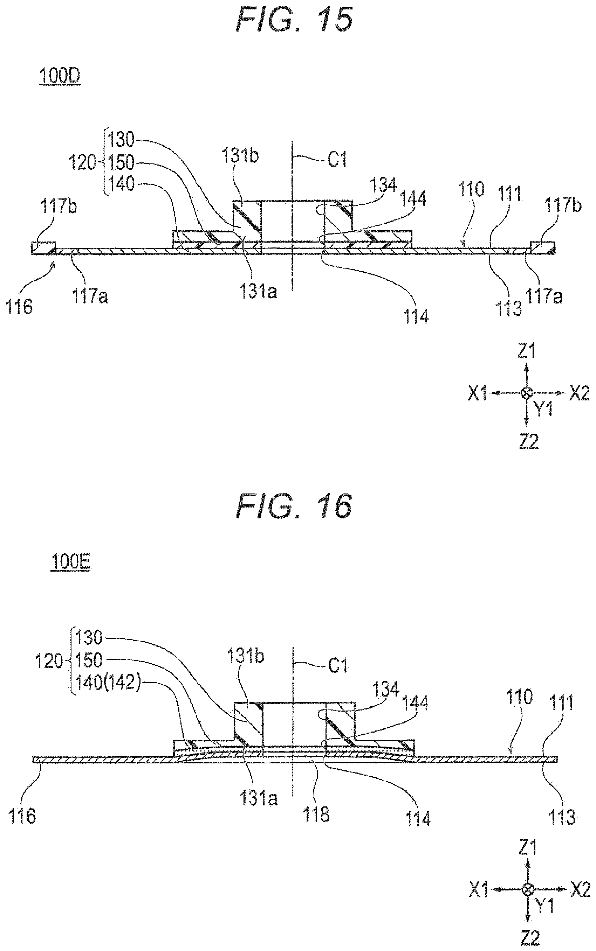

The connection portion 140 can be formed with, for example, an adhesive 141 capable of connecting the main body portion 110 and the cylindrical portion 130. However, a specific structure, material, shape, and the like, of the connection portion 140 are not particularly limited as long as the main body portion 110 and the cylindrical portion 130 can be connected to each other. For example, as will be described later, the connection portion 140 can also be formed with a welded portion 142 in which a resin material forming the cylindrical portion 130 and a biodegradable polymer forming the main body portion 110 are welded to each other (see Figs. 16 and 18).

As illustrated in Figs. 4, 5, and 6, the cylindrical portion 130 includes the edge portion 150 integrally formed with the cylindrical portion 130.

The edge portion 150 extends to the outer peripheral portion 116 side in the plane direction of the main body portion 110 from the cylindrical portion 130. Specifically, the edge portion 150 extends from the vicinity of the one end portion 131a of the cylindrical portion 130 toward the outer peripheral portion 116 side of the main body portion 110.

Note that a specific shape and size of the edge portion 150 in plan view are not particularly limited as long as at least part of the edge portion 150 extends to the outer peripheral portion 116 side of the main body portion 110 from the outer peripheral portion of the cylindrical portion 130.

For example, in a case where the edge portion 150 is formed with a resin material together with the cylindrical portion 130, the edge portion can be manufactured by integral molding using a known molding method.

The entire cylindrical portion 130 including the edge portion 150 is located on the front surface 111 side of the main body portion 110. The connection portion 140 is disposed between the edge portion 150 and the front surface 111 of the main body portion 110.

The hole portion 144 through which the first engagement portion 713 can be inserted is formed in the connection portion 140.

In the medical device 100, three regions having different physical properties are formed in a second direction from the center portion O1 side of the main body portion 110 toward the outer peripheral portion 116 side of the main body portion 110.

The above-described "second direction" is the same direction as the plane direction of the main body portion 110 in a no-load state where no external force is applied. In addition, the above-described "second direction" is synonymous with a "radial direction" of the main body portion 110 in a case where the main body portion 110 is formed in a circular planar shape. Hereinafter, the "second direction" will be also referred to as the "radial direction".

As illustrated in Figs. 3 and 6, a region (referred to as a "first axial region 115a") where the cylindrical portion 130, the edge portion 150, the connection portion 140, and the main body portion 110 overlap in the axial direction is formed at the center portion O1 of the main body portion 110 and a position surrounding the center portion O1. A region (referred to as a "second axial region 115b") in which the edge portion 150, the connection portion 140, and the main body portion 110 overlap in the axial direction is formed on the outer side in the radial direction of the first axial region 115a. A region ("third axial region 115c") in which only the main body portion 110 exists is formed on the outer side in the radial direction of the second axial region 115b.

<Relationship among hardness of cylindrical portion, hardness of connection portion, and hardness of main body portion>

The medical device 100 can be configured so that a relationship among hardness of the cylindrical portion 130, hardness of the connection portion 140, and hardness of the main body portion 110 satisfies the following expression (1) .

Hardness of cylindrical portion 130 > hardness of connection portion 140 > hardness of main body portion 110        (1)

The hardness is, for example, "Shore A hardness" specified in JIS.

When the medical device 100 is set in the first engagement tool 710, the surgeon inserts the first engagement portion 713 into the lumen 134 of the cylindrical portion 130 from the one end portion 131a side of the cylindrical portion 130 (the back surface 113 side of the main body portion 110) as illustrated in Fig. 4.

Prior to setting the medical device 100 in the first engagement tool 710, the surgeon can insert the first engagement portion 713 into a mouth side A1 of the large intestine and perform purse-string suturing in a state where the first engagement portion 713 protrudes from the mouth side A1 of the large intestine to form a suture portion A11. An outer surface of the suture portion A11 has an uneven shape as being sutured. The medical device 100 can be set in a portion of the first engagement portion 713 exposed from the suture portion A11 (see Fig 9).

When the medical device 100 is set in the first engagement tool 710, the surgeon can perform predetermined operation while gripping the cylindrical portion 130 protruding from the main body portion 110 in the first direction with fingers, tool, or the like.

As described above, in the medical device 100, the hardness of the cylindrical portion 130 is larger than the hardness of the connection portion 140 and the hardness of the main body portion 110. It is therefore possible to prevent the cylindrical portion 130 from being deformed or damaged when the surgeon grips the cylindrical portion 130 with fingers, tools, or the like. The surgeon can easily and smoothly set the medical device 100 in the first engagement tool 710 as compared with the case of handling the medical device 100 not including the cylindrical portion 130.

The surgeon can fix the medical device 100 at the first engagement portion 713 by inserting the first engagement portion 713 into the lumen 134 of the cylindrical portion 130. The surgeon can stably fix the medical device 100 at the first engagement portion 713 by maintaining a state in which the first engagement portion 713 is inserted into the lumen 134 of the cylindrical portion 130.

In the medical device 100, the hardness of the cylindrical portion 130 is larger than the hardness of the main body portion 110 as described above. Thus, in a state where the first engagement portion 713 is inserted into the lumen 134 of the cylindrical portion 130, the lumen 134 does not unintentionally expand in a radial direction of the medical device 100. Thus, the medical device 100 can maintain the inner diameter of the cylindrical portion 130 at a predetermined size before and after insertion of the first engagement portion 713. The surgeon can therefore prevent the cylindrical portion 130 from falling off from the first engagement tool 710 before starting and during performing the anastomosis.

The surgeon can maintain a state in which the first engagement portion 713 is inserted and fitted into the lumen 134 of the cylindrical portion 130 after the medical device 100 is set in the first engagement tool 710 until the anastomosis portion is formed. The surgeon can therefore prevent the medical device 100 from being displaced from the set position of the first engagement tool 710.

In the medical device 100, as described above, the hardness of the connection portion 140 connecting the cylindrical portion 130 and the main body portion 110 is smaller than the hardness of the cylindrical portion 130 and larger than the hardness of the main body portion 110. Thus, in the medical device 100, the hardness in the vicinity of the boundary where the cylindrical portion 130 and the main body portion 110 are connected gradually decreases toward the cylindrical portion 130, the connection portion 140, and the main body portion 110. The connection portion 140 moderates change in hardness between the cylindrical portion 130 and the main body portion 110 and prevents the main body portion 110 from being damaged or the cylindrical portion 130 from being separated from the main body portion 110 when stress concentration occurs near a boundary where the cylindrical portion 130 and the main body portion 110 are connected.

In the present embodiment, the cylindrical portion 130 is connected to the main body portion 110 via the edge portion 150 formed integrally with the cylindrical portion 130. As will be described later, the edge portion 150 can be configured to have the same hardness as that of the cylindrical portion 130 or hardness smaller than that of the cylindrical portion 130 and larger than that of the connection portion 140 and the main body portion 110. Thus, even in a case where the edge portion 150 is connected to the main body portion 110 via the connection portion 140, the medical device 100 can exhibit a "function of moderating the change in hardness" by the connection portion 140 described above.

<Degree of elongation and degree of contraction in first direction (axial direction)>

The medical device 100 can be configured so that, in the first direction, a relationship among a degree of elongation of the cylindrical portion 130, a degree of elongation of the connection portion 140, and a degree of elongation of the main body portion 110 satisfies the following expression (2), and
in the first direction, a relationship among a degree of contraction of the cylindrical portion 130, a degree of contraction of the connection portion 140, and a degree of contraction of the main body portion 110 satisfies the following expression (3).

Degree of elongation of cylindrical portion < degree of elongation of connection portion < degree of elongation of main body portion     (2)

Degree of contraction of cylindrical portion > degree of contraction of connection portion > degree of contraction of main body portion     (3)

The above-described "degree of elongation and the degree of contraction in the first direction" means a "maximum deformation amount" of each portion. In other words, the "degree of elongation and the degree of contraction in the first direction" is an index indicating to what extent deformation is allowed when predetermined external force (force to elongate and contract in the axial direction) is applied along the first direction from a no-load state where no external force is applied. The degree of elongation and the degree of contraction are parameters that can be adjusted by a constituent material, a wall thickness, a height, a volume, and the like, of each part as described later. The "degree of elongation and the degree of contraction in a second direction" which will be described later can also be defined in the same manner as the "degree of elongation and the degree of contraction in the first direction".

The cylindrical portion 130 has larger hardness in the first direction and a smaller degree of elongation in the first direction than the connection portion 140 and the main body portion 110. Thus, in a state where the first engagement portion 713 is inserted into the lumen 134 of the cylindrical portion 130, the cylindrical portion 130 is less likely to elongate in the axial direction when external force (external force along the axial direction) in a direction of dropping the cylindrical portion 130 from the first engagement portion 713 is applied to the fixing portion 120. As a result, when the external force along the axial direction is applied, the cylindrical portion 130 generates resistance against the first engagement portion 713 to prevent the first engagement portion 713 from coming out of the lumen 134 of the cylindrical portion 130.

Further, the cylindrical portion 130 has a larger degree of contraction in the first direction than the connection portion 140 and the main body portion 110, in addition to larger hardness in the first direction. Thus, the cylindrical portion 130 easily contracts in the axial direction when external force (external force along the first direction) in a direction of dropping the cylindrical portion 130 from the first engagement portion 713 is applied to the fixing portion 120 in a state where the first engagement portion 713 is inserted into the lumen 134 of the cylindrical portion 130. As a result, when the external force along the axial direction is applied, the cylindrical portion 130 generates resistance against the first engagement portion 713 to prevent the first engagement portion 713 from coming out of the lumen 134 of the cylindrical portion 130.

As described above, the main body portion 110 and the connection portion 140 have lower hardness and a higher degree of elongation than the cylindrical portion 130. Thus, when the first engagement portion 713 is inserted into the lumen 134 of the cylindrical portion 130, the surgeon can deform the hole portion 144 of the connection portion 140 and the vicinity of the hole portion 114 of the main body portion 110, which are inlet portions leading to the lumen 134 of the cylindrical portion 130, so as to elongate in the axial direction. Thus, the surgeon can more easily and smoothly insert the first engagement portion 713 into the lumen 134 of the cylindrical portion 130.

<Relationship among hardness of cylindrical portion, hardness of edge portion, hardness of connection portion, and hardness of main body portion>

The medical device 100 can be configured so that a relationship among the hardness of the cylindrical portion 130, the hardness of the edge portion 150, the hardness of the connection portion 140, and the hardness of the main body portion 110 satisfies the following expression (4).

Hardness of cylindrical portion $\geq$ hardness of edge portion > hardness of connection portion > hardness of main body portion     (4)

The hardness is, for example, "Shore A hardness" specified in JIS.
As described above, when the medical device 100 is set in the first engagement tool 710, the surgeon inserts the first engagement portion 713 into the lumen 134 of the cylindrical portion 130 from the one end portion 131a side of the cylindrical portion 130 (the back surface 113 side of the main body portion 110) as illustrated in Fig. 4. After part of the first engagement portion 713 is inserted into the lumen 134 of the cylindrical portion 130, the surgeon can further push the first engagement portion 713 into the lumen 134 of the cylindrical portion 130 by pushing the edge portion 150 with fingers, or the like. Thus, the surgeon can easily and reliably insert the first engagement portion 713 into the lumen 134 of the cylindrical portion 130 without directly touching the main body portion 110.

The edge portion 150 has higher hardness than the main body portion 110 and the connection portion 140. Thus, when the pushing operation as described above is performed, it is possible to transmit pushing force to the edge portion 150 firmly with fingers, or the like. Furthermore, the hardness decreases in the order of the edge portion 150, the connection portion 140, and the main body portion 110, and thus, change in hardness is gentle between the edge portion 150 and the main body portion 110. Thus, when force is applied to the edge portion 150, it is possible to prevent the main body portion 110 from being damaged near the boundary where the edge portion 150 and the main body portion 110 are connected.

The edge portion 150 may be formed to have the same hardness as the cylindrical portion 130 or may be formed to have hardness smaller than that of the cylindrical portion 130.

In the medical device 100, the edge portion 150 is disposed at a position overlapping with part of the main body portion 110 in the plane direction. Thus, in the medical device 100, an area of a flexible portion is small as compared with a case where the entire main body portion 110 is formed with a sheet-like member. Thus, deformation such as twisting hardly occurs in the main body portion 110. Thus, the medical device 100 can effectively prevent deformation such as twisting from occurring in the main body portion 110 even in a state where the medical device 100 is set in the first engagement tool 710 or in a state where the main body portion 110 is disposed so as to be in contact with the outer surface of the suture portion A11 having an uneven shape.

As illustrated in Figs. 4 and 5, the edge portion 150 is located closer to the main body portion 110 than the other end portion 131b located at the end portion on a protruding direction side of the cylindrical portion 130. Thus, the connection portion 140 connects the edge portion 150 and the main body portion 110 in a relatively wide range in which the connection portion 140 overlap the cylindrical portion 130 and the edge portion 150 in the plane direction. Accordingly, the connection portion 140 can firmly connect the cylindrical portion 130 and the edge portion 150 to the main body portion 110.

<Degree of elongation and degree of contraction in second direction (radial direction)>

The medical device 100 can be configured so that a relationship among the degree of elongation of the cylindrical portion 130, the degree of elongation of the edge portion 150, the degree of elongation of the connection portion 140, and the degree of elongation of the main body portion 110 satisfies the following expression (5) in the second direction, and in the second direction, a relationship among the degree of contraction of the cylindrical portion 130, the degree of contraction of the edge portion 150, the degree of contraction of the connection portion 140, and the degree of contraction of the main body portion 110 satisfies the following expression (6).

Degree of elongation of cylindrical portion 130 ≤ degree of elongation of edge portion 150 < degree of elongation of connection portion 140 < degree of elongation of main body portion 110　　　　(5)

Degree of contraction of cylindrical portion 130 ≥ degree of contraction of edge portion 150 > degree of contraction of connection portion 140 > degree of contraction of main body portion 110　　　　(6)

As described above, in the medical device 100, the first axial region 115a in which the cylindrical portion 130, the edge portion 150, the connection portion 140, and the main body portion 110 overlap in the axial direction, the second axial region 115b in which the edge portion 150, the connection portion 140, and the main body portion 110 overlap in the axial direction, and the third axial region 115c in which only the main body portion 110 exists are arranged in this order from the center portion O1 side toward the outer peripheral portion 116 side (that is, in the radial direction) (see Figs. 3 and 6).

When the relationship of the above expression (5) is satisfied, a magnitude relationship of the degree of elongation of the first axial region 115a, the second axial region 115b, and the third axial region 115c with respect to the second direction is "first axial region 115a ≤ second axial region 115b < third axial region 115c". Thus, in a state where the first engagement portion 713 is inserted into the lumen 134 of the cylindrical portion 130, the cylindrical portion 130 is less likely to elongate in the radial direction when external force that expands the cylindrical portion 130 in the second direction is applied to the fixing portion 120. Thus, the cylindrical portion 130 can prevent the inner diameter of the cylindrical portion 130 from expanding, so that it is possible to effectively prevent the first engagement portion 713 from coming out of the lumen 134 of the cylindrical portion 130.

When the relationship of the above expression (6) is satisfied, a magnitude relationship of the degree of contraction of the first axial region 115a, the second axial region 115b, and the third axial region 115c with respect to the second direction is "first axial region 115a $\geq$ second axial region 115b > third axial region 115c". Thus, the cylindrical portion 130 easily contracts so that the inner diameter of the cylindrical portion 130 becomes narrow in a state where the first engagement portion 713 is inserted into the lumen 134 of the cylindrical portion 130. The cylindrical portion 130 easily generates resistance with the first engagement portion 713 inserted into the lumen 134 of the cylindrical portion 130, so that it is possible to effectively prevent the first engagement portion 713 from coming out of the lumen 134 of the cylindrical portion 130.

<Constituent materials and dimensions>

As an example for satisfying the relationships of expressions (1) to (6) described above, the medical device 100 can be formed with, for example, the following materials and dimensions.

The cylindrical portion 130 can be formed with, for example, silicone elastomer. In a case where the edge portion 150 is formed integrally with the cylindrical portion 130, the edge portion 150 can be formed with the same material as the cylindrical portion 130. The constituent materials of the cylindrical portion 130 and the edge portion 150 are not limited to silicone elastomer. The cylindrical portion 130 and the edge portion 150 can be formed with, for example, another material that is harder, less stretchable, and more easily contract than the material constituting the connection portion 140.

As a material constituting the main body portion 110, for example, each of the constituent materials (for example, a biodegradable polymer) exemplified above can be used.

Physical properties (hardness, the degrees of elongation and the degrees of contraction in the first direction, the degrees of elongation and the degrees of contraction in the second direction) of the cylindrical portion 130, the edge portion 150, the connection portion 140, and the main body portion 110 described above can be arbitrarily adjusted according to the structure (thickness, height, volume, and the like) of each portion. For example, if the wall thickness D1 (tube wall thickness) of the cylindrical portion 130 is increased, an amount of the constituent material of the cylindrical portion 130 is increased, so that the hardness, the degrees of elongation, and the degrees of contraction in the first direction and the second direction can be increased. In a case where such an adjustment method is adopted, the influence on a change amount of each physical property becomes significant in the second direction (radial direction). Similarly, if a height h1 of the cylindrical portion 130 is increased, an amount of the constituent material of the cylindrical portion 130 is increased, so that the hardness, the degrees of elongation, and the degrees of contraction in the first direction can be increased. In addition, in a case where the edge portion 150 having a smaller height (thickness) than the cylindrical portion 130 is provided, an amount of the constituent material of the cylindrical portion 130 is increased as compared with the cylindrical portion 130 not including the edge portion 150, so that it is possible to mainly increase the degrees of elongation and the degrees of contraction in the second direction (radial direction).

In consideration of the above points, for example, the following dimensional examples can be adopted (see Fig. 6).

The height h1 of the cylindrical portion 130 can be formed to be, for example, 1 mm to 3 mm.

A height (thickness) h2 of the connection portion 140 can be formed to be, for example, 0.1 mm to 0.3 mm.

A height (thickness) h3 of the edge portion 150 can be formed to be, for example, 0.1 mm to 0.5 mm.

A thickness T of the main body portion 110 can be formed to be, for example, 0.05 mm to 0.3 mm.

The inner diameter d1 of the cylindrical portion 130 can be formed to be, for example, 4.8 mm to 6.6 mm. The hole diameter d0 of the hole portion 114 of the main body portion 110 and the hole diameter d2 of the hole portion 144 of the connection portion 140 can be formed to be the same as, for example, the inner diameter d1 of the cylindrical portion 130.

A wall thickness D1 of the cylindrical portion 130 can be, for example, 1 mm to 1.5 mm.

An extension length D2 of the connection portion 140 in the second direction can be set to, for example, 4 mm to 5 mm.

An extension length D3 of the edge portion 150 in the second direction can be, for example, 4 mm to 5 mm. In the medical device 100, the extension length D2 of the connection portion 140 in the second direction and the extension length D3 of the edge portion 150 in the second direction are formed to be substantially the same, but these dimensions do not have to be the same.

In the above dimensional example, the inner diameter d1 of the cylindrical portion 130 can be arbitrarily changed according to the outer diameter of the first engagement portion 713 of the first engagement tool 710 to be used for the anastomosis. For example, the inner diameter d1 of the cylindrical portion 130 is formed to be substantially the same as or smaller than the outer diameter of the first engagement portion 713, and thus, when the first engagement portion 713 is inserted into the cylindrical portion 130, the inner peripheral surface of the cylindrical portion 130 comes into close contact with the outer peripheral surface of the first engagement portion 713. It is therefore possible to more effectively prevent the cylindrical portion 130 from falling off from the first engagement portion 713.

The height h1 of the cylindrical portion 130 is more preferably 1.5 mm to 2.5 mm, and the height h2 of the edge portion 150 is more preferably 0.25 mm to 0.35 mm. This is for the following reason.

Depending on the type of the first engagement tool 710 to be used for the anastomosis, there is an engagement tool configured so that the first engagement portion 713 inserted into the lumen 134 of the cylindrical portion 130 can be inclined with respect to the axial direction of the cylindrical portion 130. If the height h1 of the cylindrical portion 130 and/or the height h3 of the edge portion 150 is formed to be excessively larger than the dimension necessary for exhibiting the function of preventing the first engagement portion 713 from falling off, the first engagement portion 713 interferes with the cylindrical portion 130 and/or the edge portion 150 when the first engagement portion 713 is inclined. As a result, a range at which the first engagement portion 713 can be inclined is limited. In consideration of such a point, the height h1 of the cylindrical portion 130 and the height h2 of the edge portion 150 are preferably the dimensions exemplified above.

<Embodiment of treatment method (colorectal anastomosis) >

Next, a treatment method using the medical device 100 will be described.

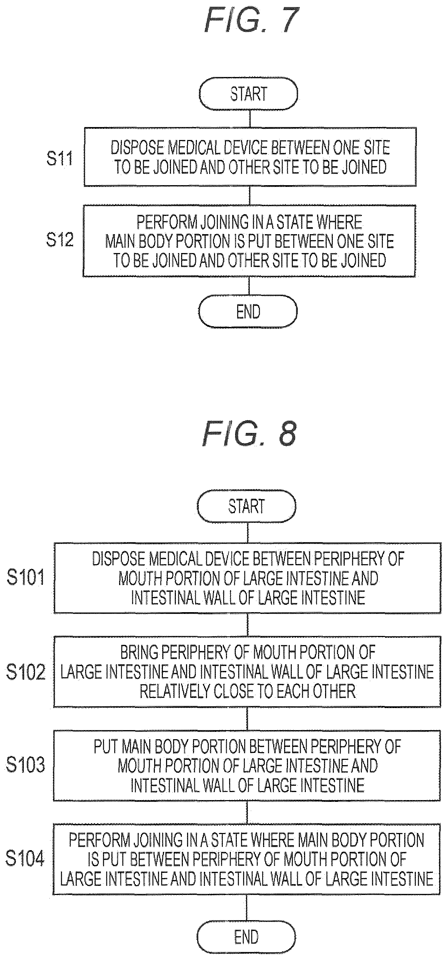

Fig. 7 is a flowchart indicating procedure of a treatment method using the medical device 100.

A treatment method includes: disposing a medical device including a main body portion that promotes fusion of living tissues at one site to be joined of a living body organ (S11); and joining the one site to be joined and the other site to be joined in a state where at least part of the main body portion of the medical device is disposed between the one site to be joined and the other site to be joined (S12).

The living body organs and the sites of the living body organs to be joined by the treatment method of the present embodiment are not particularly limited and can be arbitrarily selected. However, in the following description, large intestine anastomosis will be described as an example.

As the medical device to be used in the procedure described below, for example, a medical device having the structure illustrated in Fig. 1 can be selected. However, a specific configuration of the medical device is not particularly limited. For example, it is also possible to use a medical device of each modification described later.

In the following description, a use example of the medical device will be described as a representative example that can be suitably used for colorectal anastomosis. In the procedure described below, detailed description of a known procedure, a known medical device, a medical tool, and the like, will be appropriately omitted.

In the description of the present specification, "disposing a medical device between living body organs" means at least one of disposing the medical device in a state where the medical device is in direct or indirect contact with the living body organs, disposing the medical device in a state where a spatial gap is formed between the medical device and the living body organs, or disposing the medical device in both states (for example, the medical device is disposed in a state of being in contact with one living body organ and not in contact with the other living body organ). In addition, in the description of the present specification, the "periphery" does not define a strict range (region) and means a predetermined range (region) as long as the purpose of treatment (joining of living body organs) can be achieved. In addition, the order of the procedure described in each treatment method can be appropriately changed as long as the purpose of treatment can be achieved. In addition, in the description of the present specification, "bringing two or more objects relatively close to each other" means both bringing two or more objects close to each other and bringing only one object close to the other object.

<Embodiment of treatment method (colorectal anastomosis)>

Fig. 8 is a flowchart indicating procedure of an embodiment of a treatment method (large intestine anastomosis). Figs. 9 to 11 are views for explaining colorectal anastomosis. In Figs. 9 to 11, the medical device 100, a living body organ, and the like, are illustrated in a simplified manner.

In the treatment method according to the present embodiment, the living body organs to be joined are a large intestine cut along with resection of a cancer tumor. Specifically, the living body organs to be joined are the mouth side A1 of the cut large intestine and the anal side A2 of the cut large intestine. In the following description, procedure of joining the periphery of the mouth portion on the mouth side A1 of the cut large intestine (one site to be joined) and part of the intestinal wall on the anal side A2 of the cut large intestine (the other site to be joined) will be described.

As illustrated in Fig. 8, the treatment method according to the present embodiment includes disposing the medical device at the periphery of the mouth portion of the large intestine (S101), bringing the periphery of the mouth portion of the large intestine and the intestinal wall of the large intestine relatively close to each other (S102), putting the main body portion of the medical device between the periphery of the mouth portion of the large intestine and the intestinal wall of the large intestine (S103), and performing joining in a state where the main body portion of the medical device is put between the periphery of the mouth portion of the large intestine and the intestinal wall of the large intestine (S104).

A treatment method according to the present embodiment will be described with reference to Figs. 9 to 11.

The surgeon forms a port (an introduction portion for moving various medical tools, and the like, into and out of a living body) in the periphery of the navel of the patient, and further inflates the abdomen of the patient.

Next, the surgeon forms an incision (not illustrated) around the navel, takes out the affected part on the mouth side A1 from the incision to the outside of the body, and inserts the first engagement tool 710 of the joining device 700 into the mouth side A1 of the large intestine, as illustrated in Fig. 9. The surgeon inserts the head portion 711 of the first engagement tool 710 and the first engagement portion 713 into the mouth side A1 of the large intestine. The surgeon forms the suture portion A11 on the mouth side A1 of the large intestine.

Next, as illustrated in Fig. 9, the surgeon places the medical device 100 on the living tissues on the mouth side A1 of the large intestine (S101). When the medical device 100 is placed, the surgeon inserts the first engagement portion 713 included in the first engagement tool 710 into the lumen 134 of the cylindrical portion 130 (see Fig. 4). In this event, the surgeon can set the medical device 100 so that the back surface 113 of the main body portion 110 comes into contact with the outer surface of the suture portion A11.

Next, the surgeon introduces the mouth side A1 of the large intestine on which the medical device 100 is placed into the body of the patient from the incision.

Next, the surgeon places the second engagement tool 720 of the joining device 700 on the anal side A2 of the large intestine. As the second engagement tool 720 is placed (inserted) on the anal side A2 of the large intestine, a through hole A21 is formed in the anal side A2 of the large intestine. Note that a specific timing of forming the through hole A21 is not particularly limited.

The surgeon can place the main body portion 110 between the mouth side A1 of the large intestine and the anal side A2 of the large intestine by engaging the first engagement portion 713 of the first engagement tool 710 with the second engagement portion 723 of the second engagement tool 720 while maintaining a state where the main body portion 110 is held with respect to the mouth side A1 of the large intestine. Specifically, as illustrated in Fig. 10, the surgeon brings the first engagement tool 710 and the second engagement tool 720 relatively close to each other and engages with each other while maintaining a state where the medical device 100 is held with respect to the suture portion A11 on the mouth side A1 of the large intestine (S102).

Next, the surgeon puts the periphery of the mouth portion on the mouth side A1 of the large intestine, the main body portion 110, the fixing portion 120 disposed on the main body portion 110, and the periphery of the through hole A21 formed in the intestinal wall on the anal side A2 of the large intestine between the first engagement tool 710 and the second engagement tool 720 (S103).

The surgeon cuts part of the mouth side A1 of the large intestine put between the first engagement tool 710 and the second engagement tool 720, the main body portion 110, the fixing portion 120 disposed on the main body portion 110, and part of the anal side A2 of the large intestine by punching using the joining device 700. Furthermore, in this event, the surgeon operates the joining device 700 to join the periphery of the cut portion by stapling (not illustrated) (S104).

Next, as illustrated in Fig. 11, the surgeon takes out the joining device 700 from the anal side A2 of the large intestine to the outside of the living body through the anus, for example. In this event, the surgeon can take out part of the mouth side A1 of the large intestine located closer to the center portion O1 than the second region E2 of the main body portion 110 punched out by the joining device 700, part of the main body portion 110 of the medical device 100 (part including the fixing portion 120) and part of the anal side A2 of the large intestine together with the joining device 700 to the outside of the living body.

On the other hand, the surgeon indwells the third region E3 (see Fig. 3) of the main body portion 110 disposed closer to the outer peripheral portion 116 side than the second region E2 in the living body in a state of being put between the periphery of the mouth portion of the mouth side A1 of the large intestine and the intestinal wall on the anal side A2 of the large intestine. The indwelled portion among the main body portion 110 effectively exerts a function of promoting fusion between the periphery of the mouth portion of the mouth side A1 of the large intestine to be joined and the intestinal wall of the anal side A2 of the large intestine.

According to such a treatment method, a risk of suture failure after anastomosis (for example, colorectal anastomosis) can be reduced by a simple method of putting the main body portion 110 of the medical device 100 between one site to be joined and the other site to be joined.

(Operational effects)

As described above, the medical device 100 according to the present embodiment includes:

the sheet-like main body portion 110 in which the plurality of through holes 112 are formed, and which induces expression of the biological components by being applied to the anastomosis portion of the living body organs and promotes fusion of the anastomosis portion by allowing the induced biological components to penetrate through the through holes 112 and accumulating the induced biological components;

a cylindrical portion 130 that is disposed closer to the center portion O1 side in the plane direction of the main body portion 110 than the outer peripheral portion 116 in the plane direction of the main body portion 110, protrudes in the first direction intersecting the plane direction of the main body portion 110, and has the lumen 134 formed therein; and

the connection portion 140 that connects the main body portion 110 and the cylindrical portion 130, and

the medical device 100 is configured so that a relationship among the hardness of the cylindrical portion 130, the hardness of the connection portion 140, and the hardness of the main body portion 110 satisfies the following expression (1).

Hardness of cylindrical portion > hardness of connection portion > hardness of main body portion     (1)

According to the medical device 100 configured as described above, fusion between the living body organs can be promoted, and handleability of the sheet-like main body portion 110 at the time of use is improved.

Further, the medical device 100 is configured so that a relationship among the degree of elongation of the cylindrical portion 130, the degree of elongation of the connection portion 140, and the degree of elongation of the main body portion 110 in the first direction satisfies the following expression (2), and
the relationship among the degree of contraction of the cylindrical portion 130, the degree of contraction of the connection portion 140, and the degree of contraction of the main body portion 110 in the first direction satisfies the following expression (3),

Degree of elongation of cylindrical portion < degree of elongation of connection portion < degree of elongation of main body portion     (2)

Degree of contraction of cylindrical portion > degree of contraction of connection portion > degree of contraction of main body portion ... (3).     (3)

According to the medical device 100 configured as described above, it is possible to easily and smoothly set the joining device 700 in the first engagement tool 710. Furthermore, the medical device 100 can effectively prevent the cylindrical portion 130 from falling off from the first engagement portion 713 of the first engagement tool 710.

Further, in the medical device 100, the main body portion 110 is formed with a biodegradable polymer, and the cylindrical portion 130 is formed with a resin material. The connection portion 140 is formed with the adhesive 141.

According to the medical device 100 configured as described above, the cylindrical portion 130 and the main body portion 110 can be easily connected by the adhesive 141. In addition, by appropriately selecting the constituent material of the cylindrical portion 130 and the constituent material of the adhesive 141, the physical properties of the cylindrical portion 130 and the connection portion 140 can be adjusted to desired magnitudes.

In addition, the medical device 100 includes the edge portion 150 that is formed integrally with the cylindrical portion 130 and extends to the outer peripheral portion 116 side in the plane direction of the main body portion 110 from the cylindrical portion 130. The connection portion 140 connects the cylindrical portion 130 and/or the edge portion 150 and the main body portion 110.

According to the medical device 100 configured as described above, when the medical device 100 is set in the first engagement portion 713, the surgeon can push the edge portion 150 with fingers, or the like, to push the first engagement portion 713 into the lumen 134 of the cylindrical portion 130. Further, by connecting the edge portion 150 to the main body portion 110, the connection portion 140 can be disposed over a wider range of the main body portion 110. As a result, the cylindrical portion 130 integrally formed with the edge portion 150 can be more firmly connected to the main body portion 110.

In addition, the medical device 100 is configured so that a relationship among the hardness of the cylindrical portion 130, the hardness of the edge portion 150, the hardness of the connection portion 140, and the hardness of the main body portion 110 satisfies the following expression (4),

in the second direction from the center portion O1 side of the main body portion 110 toward the outer peripheral portion 116 side of the main body portion 110, a relationship among the degree of elongation of the cylindrical portion 130, the degree of elongation of the edge portion 150, the degree of elongation of the connection portion 140, and the degree of elongation of the main body portion 110 satisfies the following expression (5), and
a relationship among the degree of contraction of the cylindrical portion 130, the degree of contraction of the edge portion 150, the degree of contraction of the connection portion 140, and the degree of contraction of the main body portion 110 in the second direction satisfies the following expression (6).

Hardness of cylindrical portion $\geq$ hardness of edge portion > hardness of connection portion > hardness of main body portion     (4)

Degree of elongation of cylindrical portion $\leq$ degree of elongation of edge portion < degree of elongation of connection portion < degree of elongation of main body portion     (5)

Degree of contraction of cylindrical portion ≥ degree of contraction of edge portion > degree of contraction of connection portion > degree of contraction of main body portion (6)

According to the medical device 100 configured as described above, operation of setting the medical device 100 in the first engagement tool 710 of the joining device 700 can be more easily and smoothly performed. Furthermore, the medical device 100 can more effectively prevent the cylindrical portion 130 from falling off from the first engagement portion 713 of the first engagement tool 710.

In the medical device 100, the cylindrical portion 130 is disposed in a range including the center portion O1 in the plane direction of the main body portion 110.

According to the medical device 100 configured as described above, it is possible to prevent the cylindrical portion 130 (fixing portion 120) from being left in the main body portion 110 after forming the anastomosis portion in the procedure using the joining device 700.

Next, modifications of the above-described embodiment will be described. In the following description, redundant description of the content already described will be omitted. In addition, content not specifically mentioned in the following description can be the same as that in the above-described embodiment. Respective cross-sectional views illustrated in the modifications are cross-sectional views corresponding to Fig. 4 of the above-described embodiment.

(First modification)

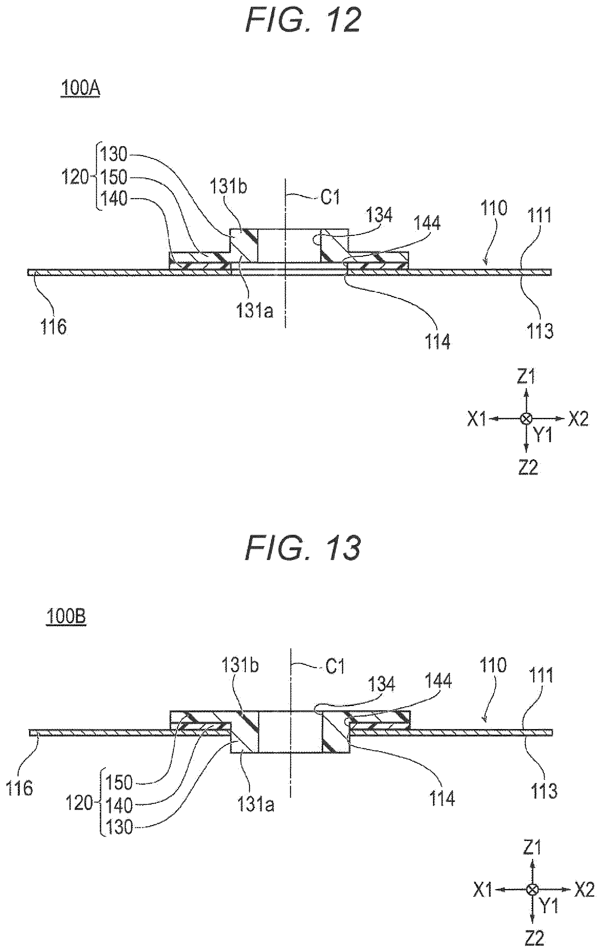

Fig. 12 illustrates a cross-sectional view of a medical device 100A according to a first modification.

As illustrated in Fig. 12, the connection portion 140 can be disposed only between the edge portion 150 and the main body portion 110. With such a configuration, the edge portion 150 and the connection portion 140 are not disposed in a portion overlapping with a portion where the cylindrical portion 130 is located in the axial direction. Thus, change in physical properties of the fixing portion 120 in the axial direction becomes further gentle. It is therefore possible to more reliably prevent the main body portion 110 from being damaged when the medical device 100A is handled.

(Second modification)

Fig. 13 illustrates a cross-sectional view of a medical device 100B according to a second modification.

As illustrated in Fig. 13, the medical device 100B according to the second modification is disposed so that the cylindrical portion 130 penetrates the main body portion 110 in a thickness direction.

One end portion 131a of the cylindrical portion 130 is disposed on the back surface 113 side of the main body portion 110, and the other end portion 131b of the cylindrical portion 130 is disposed on the front surface 111 side of the main body portion 110.

In a procedure using the medical device 100B, when the medical device 100B is set in the first engagement tool 710, a portion of the cylindrical portion 130 on the one end portion 131a side may enter inside the mouth side A1 of the large intestine (see Fig. 4) deeper than the suture portion A11.

In the medical device 100B, the edge portion 150 is disposed on the front surface 111 side of the main body portion 110. Thus, in the medical device 100B, the edge portion 150 supports the main body portion 110 on the front surface 111 side of the main body portion 110 even in a case where the portion on the one end portion 131a side of the cylindrical portion 130 enters inside the mouth side A1 of the large intestine deeper than the suture portion A11 as described above. Thus, the edge portion 150 can prevent the main body portion 110 from floating from the mouth side A1 of the large intestine or from being deformed and twisted.

(Third modification)

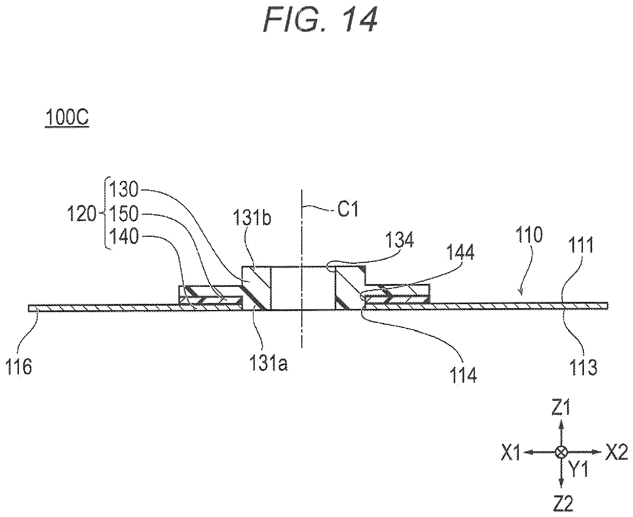

Fig. 14 illustrates a cross-sectional view of a medical device 100C according to a third modification.

As illustrated in Fig. 14, the cylindrical portion 130 can be disposed so that the one end portion 131a of the cylindrical portion 130 is located in the vicinity of the hole portion 114 of the main body portion 110. With such a configuration, in a state where the first engagement portion 713 of the first engagement tool 710 is inserted into the lumen 134 of the cylindrical portion 130, the first engagement portion 713 and the inner peripheral surface of the cylindrical portion 130 come into contact with each other in a wider range along the axial direction of the cylindrical portion 130. Thus, the cylindrical portion 130 can be more effectively prevented from falling off from the first engagement portion 713.

(Fourth modification)

Fig. 15 illustrates a cross-sectional view of a medical device 100D according to a fourth modification.

As illustrated in Fig. 15, the medical device 100D according to the fourth modification includes: a first reinforcing portion 117a that is disposed on the outer peripheral portion 116 side in the plane direction of the main body portion 110 with respect to the cylindrical portion 130 and has higher hardness than the main body portion 110; and a second reinforcing portion 117b that is disposed on the outer peripheral portion 116 side in the plane direction of the main body portion 110 with respect to the first reinforcing portion 117a and has higher hardness than the main body portion 110.

The first reinforcing portion 117a and the second reinforcing portion 117b can be disposed outside the third region E3 (see Fig. 3) to be joined to the living body organs.

The first reinforcing portion 117a can be constituted with a portion of the main body portion 110 where the through holes 112 are not formed. With such a configuration, the first reinforcing portion 117a can have higher hardness than the main body portion 110 and flexibility capable of following movement of the living body organ.

The second reinforcing portion 117b can be constituted by, for example, joining a member (for example, a resin material) harder than the main body portion 110 to the main body portion 110.

A magnitude relationship of the hardness of the main body portion 110, hardness of the first reinforcing portion 117a, and harness of the second reinforcing portion 117b can be set such that, for example, "main body portion 110 < first reinforcing portion 117a ≤ second reinforcing portion 117b". A magnitude relationship of the degrees of elongation in the second direction can be set such that, for example, "main body portion 110 > first reinforcing portion 117a ≥ second reinforcing portion 117b". Furthermore, a magnitude relationship of the degrees of contraction in the second direction can be set such that, for example, "main body portion 110 < first reinforcing portion 117a ≤ second reinforcing portion 117b". In the medical device 100D according to the present modification, the physical properties gently change in the second direction, so that it is possible to prevent the main body portion 110 from being damaged near the boundary where the first reinforcing portion 117a and the main body portion 110 are connected.

The medical device 100D includes the first reinforcing portion 117a and the second reinforcing portion 117b disposed on the outer peripheral portion 116 side of the main body portion 110, so that even in a case where the main body portion 110 is disposed in a state of being in contact with the suture portion A11 having an uneven shape, deformation such as twisting can be prevented from occurring in the main body portion 110. In addition, when the medical device 100D is handled, the surgeon can grip the first reinforcing portion 117a and the second reinforcing portion 117b with fingers. Thus, as a result of the medical device 100D including the first reinforcing portion 117a and the second reinforcing portion 117b, handleability is further improved.

(Fifth modification)

Fig. 16 illustrates a cross-sectional view of a medical device 100E according to a fifth modification.

As illustrated in Fig. 16, in the medical device 100E according to the fifth modification, the cylindrical portion 130 is connected to the front surface 111 of the main body portion 110.

The main body portion 110 has a space portion 118 formed on the back surface 113 opposite to the front surface 111 to which the cylindrical portion 130 is connected.

The space portion 118 is formed in a range overlapping with a position to which the cylindrical portion 130 is connected in plan view. The space portion 118 is recessed in a convex shape toward the front surface 111 side.

When the medical device 100E is set in the first engagement tool 710, the surgeon can dispose the medical device 100E such that part of the suture portion A11 (see Fig. 4) is accommodated in the space portion 118. As a result of the medical device 100E being disposed in this manner, the main body portion 110 can be held in a stable state with respect to the suture portion A11.

The cross-sectional shape of the space portion 118 is preferably a shape that gradually increases toward the center portion O1 side of the main body portion 110. As a result of the space portion 118 having such a shape, the suture portion A11 can be easily accommodated.

The connection portion 140 is constituted with a welded portion 142 where a resin material forming the cylindrical portion 130 and a biodegradable polymer forming the main body portion 110 are welded to each other. For example, in manufacturing process of the medical device 100E, by applying heat and pressure from the back surface 113 side of the main body portion 110 while the cylindrical portion 130 is disposed on the front surface 111 side of the main body portion 110, it is possible to form the space portion 118 recessed in a convex shape toward the front surface 111 side while part of the cylindrical portion 130 and part of the main body portion 110 are welded.

The medical device 100E manufactured by the above-described manufacturing method can be configured such that each of the cylindrical portion 130, the edge portion 150, the connection portion 140 (welded portion 142), and the main body portion 110 has desired physical properties in the first direction (axial direction) and the second direction (radial direction), in a similar manner to the medical device 100 according to the above-described embodiment. It is therefore possible to achieve effects similar to those of the medical device 100 described above.

By adjusting heat and pressure to be applied to the main body portion 110 when the connection portion 140 is formed, it is possible to gradually decrease a mixing ratio of the main body portion 110 in the connection portion 140 from the fixing portion 120 toward the main body portion 110 along the first direction. With this configuration, similarly to the medical device 100 according to the above-described embodiment, it is possible to form each of the regions 115a, 115b, and 115c (see Fig. 6) having different physical properties in the second direction (radial direction) of the medical device 100E.

Note that the cross-sectional shape of the space portion 118 is not limited to only an arc shape (shape curved toward the front surface 111 side) as illustrated in Fig. 16. The space portion 118 may have, for example, a polygonal or rectangular cross-sectional shape.

(Sixth modification)

Fig. 17 illustrates a cross-sectional view of a medical device 100F according to a sixth modification.
As illustrated in Fig. 17, in the medical device 100F, the cylindrical portion 130 does not include the edge portion 150. Thus, in the medical device 100F, the one end portion 131a of the cylindrical portion 130 is connected to the main body portion 110 via the connection portion 140. In this manner, the medical device 100F can also be constituted with the cylindrical portion 130, the connection portion 140, and the main body portion 110. In this modification, the connection portion 140 is formed with the adhesive 141.

(Seventh modification)

Fig. 18 illustrates a cross-sectional view of a medical device 100G according to a seventh modification.
In the medical device 100G, similarly to the medical device 100F according to the sixth modification described above, the cylindrical portion 130 does not include the edge portion 150. In this modification, the connection portion 140 is constituted with the welded portion 142. As described above, the medical device 100G can select any form of the connection portion 140 regardless of the presence or absence of the edge portion 150.

(Eighth modification)

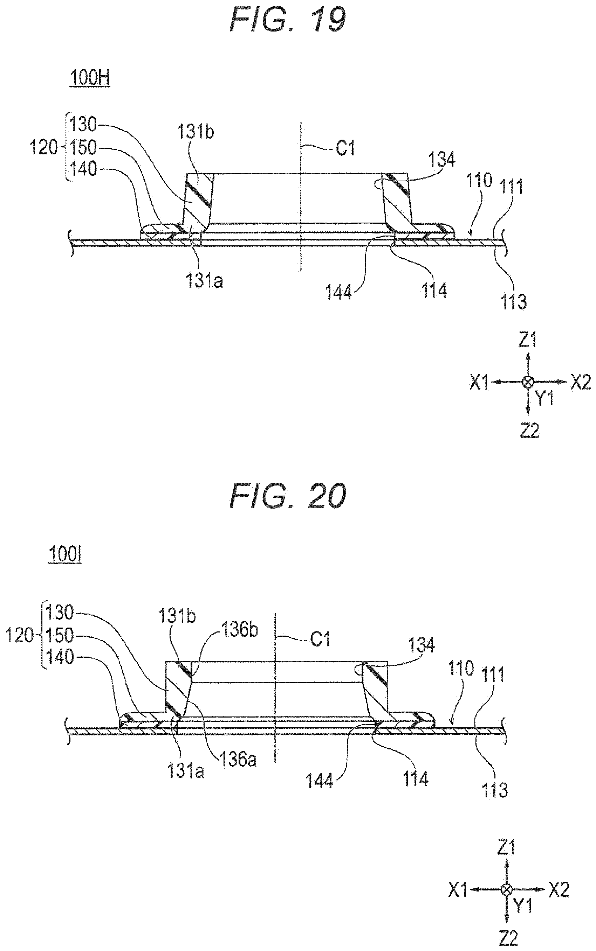

Fig. 19 illustrates a cross-sectional view of a medical device 100H according to an eighth modification.
As illustrated in Fig. 19, in the medical device 100H, the cross-sectional shape of the lumen 134 is different between the one end portion 131a located in the first direction (axial direction) of the cylindrical portion 130 and the other end portion 131b located on the opposite side of the one end portion 131a. Specifically, in the cylindrical portion 130, the one end portion 131a has a larger inner diameter than the other end portion 131b.
As illustrated in Fig. 19, the cross-sectional shape of the lumen 134 of the cylindrical portion 130 is formed such that the inner diameter gradually decreases from the one end portion 131a side toward the other end portion 131b side. Thus, when the surgeon inserts the first engagement portion 713 into the lumen 134 of the cylindrical portion 130, the surgeon can easily push the first engagement portion 713 along the axial direction of the cylindrical portion 130. In addition, the first engagement portion 713 pushed into the lumen 134 of the cylindrical portion 130 comes into contact with the inner peripheral surface of the cylindrical portion 130 on the other end portion 131b side of the cylindrical portion 130. Thus, holding force of the cylindrical portion 130 with respect to the first engagement portion 713 can be increased.

(Ninth modification)

Fig. 20 illustrates a cross-sectional view of a medical device 100I according to a ninth modification.
As illustrated in Fig. 20, a first portion 136a having an inner diameter gradually decreasing along the axial direction is formed on the one end portion 131a side of the cylindrical portion 130. A second portion 136b having a substantially constant inner diameter along the axial direction is formed on the other end portion 131b side of the cylindrical portion 130.
Similarly to the medical device 100H according to the eighth modification described above, when the first engagement portion 713 is inserted from the one end portion 131a side of the cylindrical portion 130, the medical device 100I can easily push the first engagement portion 713 along the axial direction of the cylindrical portion 130. In addition, the first engagement portion 713 pushed into the lumen 134 of the cylindrical portion 130 comes into contact with the inner peripheral surface of the cylindrical portion 130 on the other end portion 131b side of the cylindrical portion 130. The second portion 136b has a substantially constant inner diameter along the axial direction, and thus, holding force of the cylindrical portion 130 with respect to the first engagement portion 713 can be effectively increased.

(Tenth modification)

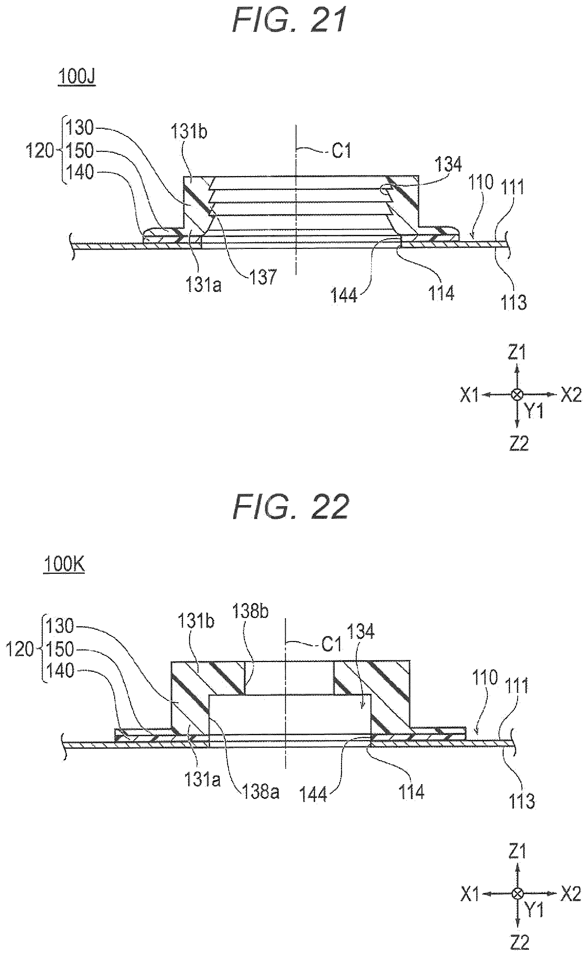

Fig. 21 illustrates a cross-sectional view of a medical device 100J according to a tenth modification.

As illustrated in Fig. 21, the one end portion 131a of the cylindrical portion 130 has an inner diameter larger than that of the other end portion 131b of the cylindrical portion 130. A plurality of inclined surfaces 137 inclined toward the other end portion 131b side is formed on the inner peripheral surface of the cylindrical portion 130. When the surgeon inserts the first engagement portion 713 into the lumen 134 of the cylindrical portion 130 from the one end portion 131a side of the cylindrical portion 130, the first engagement portion 713 smoothly moves along the inclined surfaces 137. Thus, the surgeon can easily push the first engagement portion 713 along the axial direction of the cylindrical portion 130. In addition, in a state where the first engagement portion 713 is inserted into the lumen 134 of the cylindrical portion 130, each of the inclined surfaces 137 generates resistance to prevent movement of the first engagement portion 713 toward the one end portion 131a. Thus, the medical device 100J can effectively increase holding force of the cylindrical portion 130 with respect to the first engagement portion 713.

(Eleventh modification)

Fig. 22 illustrates a cross-sectional view of a medical device 100K according to an eleventh modification.

As illustrated in Fig. 22, a first portion 138a having a constant inner diameter along the axial direction is formed on the one end portion 131a side of the cylindrical portion 130. A second portion 138b that is smaller than the first portion 138a and has a constant inner diameter along the axial direction is formed on the other end portion 131b side of the cylindrical portion 130.

When the surgeon inserts the first engagement portion 713 from the one end portion 131a side of the cylindrical portion 130, the first engagement portion 713 can be easily inserted from the vicinity of the one end portion 131a having a large inner diameter. In addition, the first engagement portion 713 pushed into the lumen 134 of the cylindrical portion 130 comes into contact with the inner peripheral surface of the cylindrical portion 130 on the other end portion 131b side of the cylindrical portion 130. Thus, holding force of the cylindrical portion 130 with respect to the first engagement portion 713 can be increased.

Although the medical device according to the present invention has been described above through the embodiment and the modifications, the present invention is not limited only to the content described in the embodiment and the modifications and can be appropriately changed on the basis of the description of the claims.

The living body organs to be joined, sites to be joined, a specific procedure, and the like, are not limited to those described in the embodiment.

A material, a size, an outer shape, a cross-sectional shape, a specific structure, and the like, of each portion of the medical device are not particularly limited as long as the main body portion included in the medical device has a function of promoting fusion of living tissues of living body organs.

The structures described in the embodiment and the modifications can be arbitrarily and selectively combined as long as the respective functions are not impaired.

The present application is based on Japanese Patent Application No. 2021-156611 filed on September 27, 2021.

Reference Signs List

100, 100A, 100B, 100C, 100D, 100E, 100F, 100G, 100H, 100I, 100J, 100K Medical device
110 Main body portion
111 Front surface of main body portion
112 Through hole
113 Back surface of main body portion
114 Hole portion
115a First axial region
115b Second axial region
115c Third axial region
116 Outer peripheral portion
117a First reinforcing portion
117b Second reinforcing portion
118 Space portion
120 Fixing portion
130 Cylindrical portion
131a One end portion of cylindrical portion
131b Other end portion of cylindrical portion
134 Lumen

140 Connection portion
141 Adhesive
142 Welded portion
144 Hole portion
150 Edge portion
700 Joining device
710 First engagement tool
711 Head portion
713 First engagement portion
720 Second engagement tool
723 Second engagement portion
A1 Mouth side
A11 Suture portion
A2 Anal side
A21 Through hole
O1 Center portion of main body portion

**Claims**

1. A medical device (100, 100A-K) comprising:

a sheet-like main body portion (110) in which a plurality of through holes (112, A21) are formed, and which induces expression of biological components by being applied to an anastomosis portion of living body organs and promotes fusion of the anastomosis portion by allowing the induced biological components to penetrate through the through holes (112, A21) and accumulating the induced biological components;
a cylindrical portion (130) that is disposed closer to a center portion side in a plane direction of the main body portion (110) than an outer peripheral portion (116) in the plane direction of the main body portion (110), protrudes in a first direction intersecting the plane direction of the main body portion (110), and has a lumen (134) formed therein; and
a connection portion (140) that connects the main body portion (110) and the cylindrical portion,
wherein a relationship among hardness of the cylindrical portion (130), hardness of the connection portion (140), and hardness of the main body portion (110) satisfies the following expression (1):

Hardness of cylindrical portion > hardness of connection portion > hardness of main Body portion　　　(1)

wherein the medical device further comprises an edge portion (150) that is formed integrally with the cylindrical portion (130) and extends to an outer peripheral portion side in the plane direction of the main body portion (110) with respect to the cylindrical portion (130),
wherein the connection portion (140) connects the cylindrical portion (130) and/or the edge portion (150) and the main body portion (110).

2. The medical device (100, 100A-K) according to claim 1,

wherein a relationship among a degree of elongation of the cylindrical portion (130), a degree of elongation of the connection portion (140), and a degree of elongation of the main body portion (110) in the first direction satisfies the following expression (2): and
a relationship among a degree of contraction of the cylindrical portion (130), a degree of contraction of the connection portion (140), and a degree of contraction of the main body portion (110) in the first direction satisfies the following expression (3):

Degree of elongation of cylindrical portion (130) < degree of elongation of connection portion (140) < degree of elongation of main body portion (110),　　　(2)

Degree of contraction of cylindrical portion (130) > degree of contraction of connection portion (140) > degree of contraction of main body portion (110).　　　(3)

3. The medical device (100, 100A-K) according to claim 1 or 2,

wherein the main body portion (110) is formed with a biodegradable polymer,
the cylindrical portion (130) is formed with a resin material, and
the connection portion (140) is constituted with a welded portion (142) in which an adhesive (141) or the resin material forming the cylindrical portion (130) and the biodegradable polymer forming the main body portion (110) are welded.

4. The medical device (100, 100A-K) according to any one of claims 1 to 3,
wherein a relationship among hardness of the cylindrical portion (130), hardness of the edge portion (150), hardness of the connection portion (140), and hardness of the main body portion (110) satisfies the following expression (4):

in a second direction from a center portion side of the main body portion (110) toward an outer peripheral portion side of the main body portion (110), a relationship among a degree of elongation of the cylindrical portion (130), a degree of elongation of the edge portion (150), a degree of elongation of the connection portion (140), and a degree of elongation of the main body portion (110) satisfies the following expression (5): and
a relationship among a degree of contraction of the cylindrical portion (130), a degree of contraction of the edge portion (150), a degree of contraction of the connection portion (140), and a degree of contraction of the main body portion (110) in the second direction satisfies the following expression (6):

Hardness of cylindrical portion (130) ≥ hardness of edge portion (150) > hardness of connection portion (140) > hardness of main body portion (110) $\qquad$ (4)

Degree of elongation of cylindrical portion (130) ≤ degree of elongation of edge portion (150) < degree of elongation of connection portion (140) < degree of elongation of main body portion (110) $\qquad$ (5)

Degree of contraction of cylindrical portion (130) ≥ degree of contraction of edge portion (150) > degree of contraction of connection portion (140) > degree of contraction of main body portion (110). $\qquad$ (6)

5. The medical device (100, 100A-K) according to any one of claims 1 to 4, wherein
the cylindrical portion (130) is disposed in a range including a center portion (O1) of the main body portion (110) in the plane direction.

6. The medical device (100, 100A-K) according to any one of claims 1 to 5,

wherein a first reinforcing portion (117a) that is disposed closer to an outer peripheral portion (116) in the plane direction of the main body portion (110) than the cylindrical portion (130) and has hardness higher than the hardness of the main body portion (110); and
a second reinforcing portion (117b) that is disposed closer to the outer peripheral portion (116) in the plane direction of the main body portion (110) than the first reinforcing portion (117a) and has hardness higher than the hardness of the main body portion (110).

7. The medical device (100, 100A-K) according to any one of claims 1 to 6,

wherein the cylindrical portion (130) is connected to one of a front surface and a back surface of the main body portion (110), and
a space portion (118) recessed in a convex shape toward the one surface side is formed on the other surface side opposite to the one surface to which the cylindrical portion (130) is connected in a range overlapping with a position to which the cylindrical portion (130) is connected in plan view.

8. The medical device (100, 100A-K) according to any one of claims 1 to 7,
wherein a cross-sectional shape of a lumen (134) of the cylindrical portion (130) is different between one end portion (131a) of the cylindrical portion (130) located along the first direction and the other end portion (131b) located on an opposite side of the one end portion, and the one end portion (131a) has a larger inner diameter than the other end portion (131b).

**Patentansprüche**

1.  Medizinische Vorrichtung (100, 100A-K), umfassend:

    einen flächenförmigen Hauptkörperabschnitt (110), in dem eine Vielzahl von Durchgangslöchern (112, A21) ausgebildet ist und der die Expression biologischer Komponenten induziert, indem er auf einen Anastomose-Abschnitt von Organen eines lebenden Körpers aufgebracht wird, und die Fusion des Anastomose-Abschnitts fördert, indem er die induzierten biologischen Komponenten durch die Durchgangslöcher (112, A21) eindringen lässt und die induzierten biologischen Komponenten ansammelt;
    einen zylindrischen Abschnitt (130), der in einer Ebenenrichtung des Hauptkörperabschnitts (110) näher an einer Mittelabschnittsseite angeordnet ist als ein äußerer Umfangsabschnitt (116) in der Ebenenrichtung des Hauptkörperabschnitts (110), in einer ersten Richtung hervorsteht, welche die Ebenenrichtung des Hauptkörperabschnitts (110) schneidet, und ein Lumen (134) aufweist, das darin ausgebildet ist; und einen Verbindungsabschnitt (140), der den Hauptkörperabschnitt (110) und den zylindrischen Abschnitt verbindet,
    wobei eine Beziehung zwischen der Härte des zylindrischen Abschnitts (130), der Härte des Verbindungsabschnitts (140) und der Härte des Hauptkörperabschnitts (110) die folgende Gleichung (1) erfüllt:

    $$\text{Härte des zylindrischen Abschnitts} > \text{Härte des Verbindungsabschnitts} > \text{Härte des Hauptkörperab-schnitts} \tag{1}$$

    wobei die medizinische Vorrichtung ferner einen Randabschnitt (150) umfasst, der einstückig mit dem zylindrischen Abschnitt (130) ausgebildet ist und sich zu einer äußeren Umfangsseite in der Ebenenrichtung des Hauptkörperabschnitts (110) in Bezug auf den zylindrischen Abschnitt (130) erstreckt,
    wobei der Verbindungsabschnitt (140) den zylindrischen Abschnitt (130) und/oder den Randabschnitt (150) und den Hauptkörperabschnitt (110) verbindet.

2.  Medizinische Vorrichtung (100, 100A-K) nach Anspruch 1, wobei eine Beziehung zwischen einem Grad der Dehnung des zylindrischen Abschnitts (130), einem Grad der Dehnung des Verbindungsabschnitts (140) und einem Grad der Dehnung des Hauptkörperabschnitts (110) in der ersten Richtung die folgende Gleichung (2) erfüllt; und eine Beziehung zwischen einem Grad der Kontraktion des zylindrischen Abschnitts (130), einem Grad der Kontraktion des Verbindungsabschnitts (140) und einem Grad der Kontraktion des Hauptkörperabschnitts (110) in der ersten Richtung die folgende Gleichung (3) erfüllt:

    $$\text{Grad der Dehnung des zylindrischen Abschnitts (130)} < \text{Grad der Dehnung des Verbindungsabschnitts (140)} < \text{Grad der Dehnung des Hauptkörperabschnitts (110)}, \tag{2}$$

    $$\text{Grad der Kontraktion des zylindrischen Abschnitts (130)} > \text{Grad der Kontraktion des Verbindungsab-schnitts (140)} > \text{Grad der Kontraktion des Hauptkörperabschnitts (110)}. \tag{3}$$

3.  Medizinische Vorrichtung (100, 100A-K) nach Anspruch 1 oder 2,

    wobei der Hauptkörperabschnitt (110) aus einem biologisch abbaubaren Polymer gebildet ist,
    der zylindrische Abschnitt (130) aus einem Harzmaterial gebildet ist, und
    der Verbindungsabschnitt (140) mit einem Schweißabschnitt (142) gebildet ist, in dem ein Klebemittel (141) oder das Harzmaterial, das den zylindrischen Abschnitt (130) bildet, und das biologisch abbaubare Polymer, das den Hauptkörperabschnitt (110) bildet, verschweißt sind.

4.  Medizinische Vorrichtung (100, 100A-K) nach einem der Ansprüche 1 bis 3,
    wobei eine Beziehung zwischen der Härte des zylindrischen Abschnitts (130), der Härte des Randabschnitts (150), der Härte des Verbindungsabschnitts (140) und der Härte des Hauptkörperabschnitts (110) die folgende Gleichung (4) erfüllt:

in einer zweiten Richtung von einer Mittelabschnittsseite des Hauptkörperabschnitts (110) zu einer äußeren Umfangsabschnittseite des Hauptkörperabschnitts (110) eine Beziehung zwischen einem Grad der Dehnung des zylindrischen Abschnitts (130), einem Grad der Dehnung des Randabschnitts (150), einem Grad der Dehnung des Verbindungsabschnitts (140) und einem Grad der Dehnung des Hauptkörperabschnitts (110) die folgende Beziehung (5) erfüllt: und

eine Beziehung zwischen einem Grad der Kontraktion des zylindrischen Abschnitts (130), einem Grad der Kontraktion des Randabschnitts (150), einem Grad der Kontraktion des Verbindungsabschnitts (140) und einem Grad der Kontraktion des Hauptkörperabschnitts (110) in der zweiten Richtung die folgende Gleichung (6) erfüllt:

$$\text{Härte des zylindrischen Abschnitts (130)} \geq \text{Härte des Randabschnitts (150)} > \text{Härte des Verbindungsabschnitts (140)} > \text{Härte des Hauptkörperabschnitts (110)} \tag{4}$$

$$\text{Grad der Dehnung des zylindrischen Abschnitts (130)} \leq \text{Grad der Dehnung des Randabschnitts (150)} < \text{Grad der Dehnung des Verbindungsabschnitts (140)} < \text{Grad der Dehnung des Hauptkörperabschnitts (110)} \tag{5}$$

$$\text{Grad der Kontraktion des zylindrischen Abschnitts (130)} \geq \text{Grad der Kontraktion des Randabschnitts (150)} > \text{Grad der Kontraktion des Verbindungsabschnitts (140)} > \text{Grad der Kontraktion des Hauptkörperabschnitts (110)}. \tag{6}$$

5. Medizinische Vorrichtung (100, 100A-K) nach einem der Ansprüche 1 bis 4, wobei der zylindrische Abschnitt (130) in einem Bereich angeordnet ist, der einen Mittelabschnitt (O1) des Hauptkörperabschnitts (110) in der Ebenenrichtung umfasst.

6. Medizinische Vorrichtung (100, 100A-K) nach einem der Ansprüche 1 bis 5,

wobei ein erster Verstärkungsabschnitt (117a), der in der Ebenenrichtung des Hauptkörperabschnitts (110) näher an einem äußeren Umfangsabschnitt (116) angeordnet ist als der zylindrische Abschnitt (130) und eine Härte aufweist, die höher ist als die Härte des Hauptkörperabschnitts (110); und
ein zweiter Verstärkungsabschnitt (117b), der in der Ebenenrichtung des Hauptkörperabschnitts (110) näher an dem äußeren Umfangsabschnitt (116) angeordnet ist als der erste Verstärkungsabschnitt (117a), und eine Härte aufweist, die höher ist als die Härte des Hauptkörperabschnitts (110).

7. Medizinische Vorrichtung (100, 100A-K) nach einem der Ansprüche 1 bis 6,

wobei der zylindrische Abschnitt (130) mit einer von einer Vorderseite und einer Rückseite des Hauptkörperabschnitts (110) verbunden ist, und
ein Raumabschnitt (118), der in einer konvexen Form in Richtung zu der einen Oberflächenseite hin vertieft ist, ist auf der anderen Oberflächenseite gegenüber der einen Oberfläche, mit welcher der zylindrische Abschnitt (130) verbunden ist, in einem Bereich ausgebildet, der sich mit einer Position überlappt, mit welcher der zylindrische Abschnitt (130) in der Draufsicht verbunden ist.

8. Medizinische Vorrichtung (100, 100A-K) nach einem der Ansprüche 1 bis 7, wobei eine Querschnittsform eines Lumens (134) des zylindrischen Abschnitts (130) zwischen einem Endabschnitt (131a) des zylindrischen Abschnitts (130), der entlang der ersten Richtung angeordnet ist, und dem anderen Endabschnitt (131b), der auf einer gegenüberliegenden Seite des einen Endabschnitts angeordnet ist, unterschiedlich ist, und der eine Endabschnitt (131a) einen größeren Innendurchmesser als der andere Endabschnitt (131b) aufweist.

## Revendications

1. Dispositif médical (100, 100A-K) comprenant :

une partie de corps principal semblable à une feuille (110) dans laquelle une pluralité de trous traversants (112, A21) sont formés, et qui induit l'expression de composants biologiques en étant appliqué sur une partie d'anastomose d'organes d'organismes vivants et favorise la fusion de la partie d'anastomose en permettant aux composants biologiques induits de pénétrer à travers les trous traversants (112, A21) et en accumulant les composants biologiques induits ;

une partie cylindrique (130) qui est disposée plus près d'un côté d'une partie centrale dans une direction plane de la partie de corps principal (110) qu'une partie périphérique extérieure (116) dans la direction plane de la partie de corps principal (110), fait saillie dans une première direction coupant la direction plane de la partie de corps principal (110), et dans laquelle une lumière (134) est formée ; et

une partie de connexion (140) qui connecte la partie de corps principal (110) et la partie cylindrique,

dans lequel une relation entre la dureté de la partie cylindrique (130), la dureté de la partie de connexion (140) et la dureté de la partie de corps principal (110) répond à l'expression (1) suivante :

Dureté de la partie cylindrique > dureté à la partie de connexion > dureté de la partie de corps principal  (1)

dans lequel le dispositif médical comprend en outre une partie de bord (150) qui est formée intégralement avec la partie cylindrique (130) et s'étend vers un côté de partie périphérique extérieure dans la direction plane de la partie de corps principal (110) par rapport à la partie cylindrique (130),

dans lequel la partie de connexion (140) connecte la partie cylindrique (130) et/ou la partie de bord (150) et la partie de corps principal (110).

2. Dispositif médical (100, 100A-K) selon la revendication 1,

dans lequel une relation entre un degré d'élongation de la partie cylindrique (130), un degré d'élongation de la partie de connexion (140) et un degré d'élongation de la partie de corps principal (110) dans la première direction répond à l'expression (2) suivante ; et

une relation entre un degré de contraction de la partie cylindrique (130), un degré de contraction de la partie de connexion (140) et un degré de contraction de la partie de corps principal (110) dans la première direction répond à l'expression (3) suivante :

Degré d'élongation de la partie cylindrique (130) < degré d'élongation de la partie de connexion (140) < degré d'élongation de la partie de corps principal (110),  (2)

Degré de contraction de la partie cylindrique (130) > degré de contraction de la partie de connexion (140) > degré de contraction de la partie de corps principal (110).  (3)

3. Dispositif médical (100, 100A-K) selon la revendication 1 ou 2,

dans lequel la partie de corps principal (110) est formée à partir d'un polymère biodégradable,

la partie cylindrique (130) est formée à partir d'une résine, et

la partie de connexion (140) est constituée d'une partie soudée (142) dans laquelle un adhésif (141) ou la résine formant la partie cylindrique (130) et le polymère biodégradable formant la partie de corps principal (110) sont soudés.

4. Dispositif médical (100, 100A-K) selon l'une quelconque des revendications 1 à 3,

dans lequel une relation entre la dureté de la partie cylindrique (130), la dureté de la partie de bord (150), la dureté de la partie de connexion (140) et la dureté de la partie de corps principal (110) répond à l'expression (4) suivante :

dans une seconde direction depuis un côté d'une partie centrale de la partie de corps principal (110) vers un côté d'une partie périphérique extérieure de la partie de corps principal (110), une relation entre un degré d'élongation de la partie cylindrique (130), un degré d'élongation de la partie de bord (150), un degré d'élongation de la partie de connexion (140) et un degré d'élongation de la partie de corps principal (110) répond à l'expression (5) suivante : et

une relation entre un degré de contraction de la partie cylindrique (130), un degré de contraction de la partie de bord (150), un degré de contraction de la partie de connexion (140) et un degré de contraction de la partie de corps principal (110) dans la seconde direction répond à l'expression (6) suivante :

Dureté de la partie cylindrique (130) ≥ dureté de la partie de bord (150) > dureté de la partie de connexion (140) > dureté de la partie de corps principal (110)

(4)

Degré d'élongation de la partie cylindrique (130) ≤ degré d'élongation de la partie de bord (150) < degré d'élongation de la partie de connexion (140) < degré d'élongation de la partie de corps principal (110)

(5)

Degré de contraction de la partie cylindrique (130) ≥ degré de contraction de la partie de bord (150) > degré de contraction de la partie de connexion (140) > degré de contraction de la partie de corps principal (110).

(6)

5. Dispositif médical (100, 100A-K) selon l'une quelconque des revendications 1 à 4, dans lequel la partie cylindrique (130) est disposée dans une zone comprenant une partie centrale (O1) de la partie de corps principal (110) dans la direction plane.

6. Dispositif médical (100, 100A-K) selon l'une quelconque des revendications 1 à 5,

dans lequel une première partie de renforcement (117a) est disposée plus près d'une partie périphérique extérieure (116) dans la direction plane de la partie de corps principal (110) que la partie cylindrique (130) et a une dureté supérieure à la dureté de la partie de corps principal (110) ; et
une seconde partie de renforcement (117b) est disposée plus près de la partie périphérique extérieure (116) dans la direction plane de la partie de corps principal (110) que la première partie de renforcement (117a) et a une dureté supérieure à la dureté de la partie de corps principal (110).

7. Dispositif médical (100, 100A-K) selon l'une quelconque des revendications 1 à 6,

dans lequel la partie cylindrique (130) est connectée à l'une d'une surface avant et d'une surface arrière de la partie de corps principal (110), et
une partie d'espacement (118) renfoncée en une forme convexe vers l'un côté de surface est formée sur l'autre côté de surface opposé à l'une surface à laquelle la partie cylindrique (130) est connectée en une zone recouvrant une position à laquelle la partie cylindrique (130) est connectée dans une vue en plan.

8. Dispositif médical (100, 100A-K) selon l'une quelconque des revendications 1 à 7,
dans lequel une forme transversale d'une lumière (134) de la partie cylindrique (130) est différente entre une partie d'extrémité (131a) de la partie cylindrique (130) située le long de la première direction et l'autre partie d'extrémité (131b) située sur un côté opposé de l'une partie d'extrémité, et l'une partie d'extrémité (131a) a un diamètre intérieur supérieur à celui de l'autre partie d'extrémité (131b).

# FIG. 1

# FIG. 2

# FIG. 3

# FIG. 4

## FIG. 5

## FIG. 6

## FIG. 7

START

S11 | DISPOSE MEDICAL DEVICE BETWEEN ONE SITE TO BE JOINED AND OTHER SITE TO BE JOINED

S12 | PERFORM JOINING IN A STATE WHERE MAIN BODY PORTION IS PUT BETWEEN ONE SITE TO BE JOINED AND OTHER SITE TO BE JOINED

END

## FIG. 8

START

S101 | DISPOSE MEDICAL DEVICE BETWEEN PERIPHERY OF MOUTH PORTION OF LARGE INTESTINE AND INTESTINAL WALL OF LARGE INTESTINE

S102 | BRING PERIPHERY OF MOUTH PORTION OF LARGE INTESTINE AND INTESTINAL WALL OF LARGE INTESTINE RELATIVELY CLOSE TO EACH OTHER

S103 | PUT MAIN BODY PORTION BETWEEN PERIPHERY OF MOUTH PORTION OF LARGE INTESTINE AND INTESTINAL WALL OF LARGE INTESTINE

S104 | PERFORM JOINING IN A STATE WHERE MAIN BODY PORTION IS PUT BETWEEN PERIPHERY OF MOUTH PORTION OF LARGE INTESTINE AND INTESTINAL WALL OF LARGE INTESTINE

END

# FIG. 9

# FIG. 10

# FIG. 11

## FIG. 12

100A

## FIG. 13

100B

FIG. 14

100C

## FIG. 15

100D

## FIG. 16

100E

## FIG. 17

100F

## FIG. 18

100G

FIG. 19

100H

130
120 150     131b
140                    C1                    134
                                                      110  111

131a                    144        114

                                           113

Z1
X1 ⊗ X2
   Y1
Z2

FIG. 20

100I

130      131b
120 150          136b      C1
140                              134      110  111

131a  136a      144    114

                              113

Z1
X1 ⊗ X2
   Y1
Z2

## FIG. 21

100J

## FIG. 22

100K

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007505708 A
- WO 2019156230 A
- JP 2011528275 W
- JP 2008514719 W
- WO 20081952 A
- JP 2004509205 W
- JP 2021156611 A